(19) Europäisches Patentamt
European Patent Office
Office européen des brevets

(11) Veröffentlichungsnummer: **0 410 929 A2**

(12) # EUROPÄISCHE PATENTANMELDUNG

(21) Anmeldenummer: **90810545.5**

(51) Int. Cl.[5]: **C08G 65/40**, C07C 43/00

(22) Anmeldetag: **17.07.90**

(30) Priorität: **24.07.89 CH 2762/89**

(43) Veröffentlichungstag der Anmeldung:
**30.01.91 Patentblatt 91/05**

(84) Benannte Vertragsstaaten:
**DE ES FR GB IT NL SE**

(71) Anmelder: **CIBA-GEIGY AG**
**Klybeckstrasse 141**
**CH-4002 Basel(CH)**

(72) Erfinder: **Pfaendner, Rudolf, Dr.**
**Sackgasse 3**
**D-6149 Rimbach/Odenwald 1(DE)**
Erfinder: **Kainmüller, Thomas, Dr.**
**Am Kümmelberg 1**
**D-6145 Lindenfels/Odenwald(DE)**
Erfinder: **Hoffmann, Kurt, Dr.**
**Heidenbergstrasse 15**
**D-6147 Lautertal 2(DE)**
Erfinder: **Wolf, Jean-Pierre, Dr.**
**Route du Couchant 1**
**CH-1723 Marly(CH)**
Erfinder: **Kramer, Andreas, Dr.**
**Bundtels**
**CH-3186 Düdingen(CH)**
Erfinder: **Stockinger, Friedrich**
**Au Fernotz**
**CH-1784 Courtepin(CH)**

(54) **Lineare Polymere.**

(57) Beschrieben werden im wesentlichen lineare Polymere, die durch die Anwesenheit eines Restes der Formel

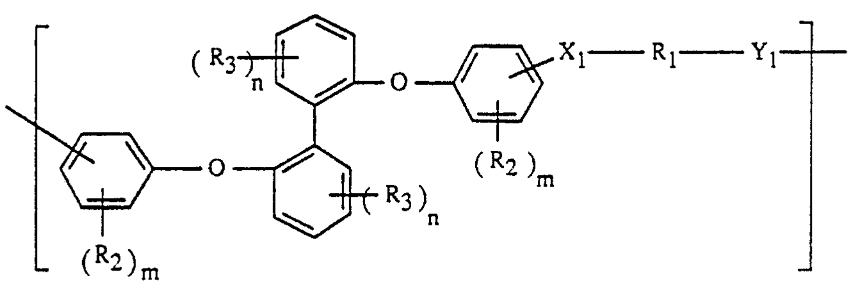

gekennzeichnet sind, worin $X_1$ ausgewählt wird aus der Gruppe bestehend aus -O-, -O-$SO_2$-, -O-CO-, -O-CO-O-, und -O-CO-NH-, $Y_1$ ausgewählt wird aus der Gruppe bestehend aus -O-, -$SO_2$-O-,-CO-O-, -O-CO-O- und -NH-CO-O-, $R_1$ einen zweiwertigen aliphatischen, cycloaliphatischen, aromatischen oder araliphatischen Rest nach dem Entfernen der funktionellen Gruppen bedeutet, oder worin die Gruppe $X_1$-$R_1$ für den Fall, dass $Y_1$ -O- oder -O-CO-O- bedeutet, auch eine direkte C-C-Bindung sein kann, $R_2$ Alkyl, Alkenyl, Cycloalkyl, Aryl oder Halogen bedeutet, $R_3$ Alkyl, Cycloalkyl, Aryl oder Halogen ist, und m und n unabhängig voneinander ganze Zahlen von 0 bis 4 darstellen.

EP 0 410 929 A2

# LINEARE POLYMERE

Die vorliegende Erfindung betrifft im wesentlichen lineare Polymere enthaltend eine neue Bisphenoleinheit, sowie die neuen Bisphenole.

Polymere, insbesondere thermoplastische Polymere mit überwiegend aromatischen Gruppen, sind häufig in herkömmlichen organischen Lösungsmitteln, wie chlorierten Kohlenwasserstoffen, schwer oder überhaupt nicht löslich. So sind beispielsweise die bislang bekannten Polyarylenetherketone in der Regel in solchen Lösungsmitteln praktisch unlöslich.

Beispiele für solche Polymere sind die aus der EP-A-1,879 bekannten Polyetherketone auf Basis von Hydrochinon und 4,4'-Difluorbenzophenon.

Aus der US-A-3,875,103 ist eine Beschichtungslösung für Polyethersulfone bekannt. Die Lösung erfordert ausgewählte Lösungsmittel und ein ausgewähltes Mischungsverhältnis der Komponenten.

Es gibt Anwendungen, bei denen eine möglichst gute Löslichkeit der Polymeren erwünscht ist. So wird beispielsweise bei der Modifizierung von duromeren Matrixharzen mit thermoplastischen Polymeren im allgemeinen mit möglichst konzentrierten Lösungen dieser Polymeren in herkömmlichen organischen Lösungsmitteln gearbeitet. Die Lösungsmittel sollen nach dem Einarbeiten rasch entfernbar sein und deshalb möglichst niedrige Verdampfungspunkte aufweisen.

Es wurden jetzt Polymere gefunden, die sich durch eine gute Löslichkeit in einer Reihe von Lösungsmitteln auszeichnen und mit denen sich Lösungen mit verbesserter Lagerstabilität herstellen lassen.

Die vorliegende Erfindung betrifft im wesentlichen lineare Polymere mit einer reduzierten Viskosität von etwa 0,1 bis etwa 2,0 dl/g (gemessen an einer 1 Gew. %igen Lösung in NMP bei 25°C) enthaltend mindestens einen die Löslichkeit des Polymeren erhöhenden Anteil an Struktureinheiten der Formel I

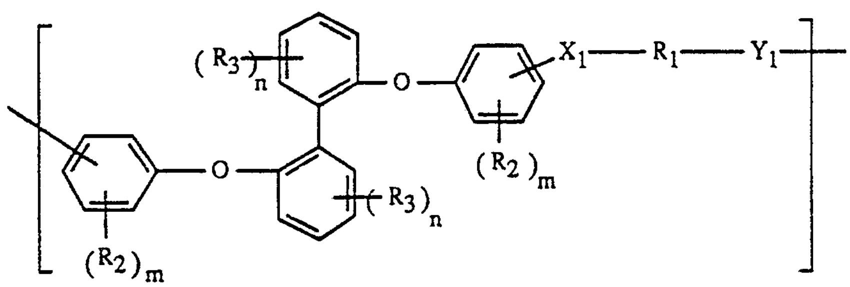

(I),

worin $X_1$ ausgewählt wird aus der Gruppe bestehend aus -O-, $-O-SO_2-$, -O-CO-, -O-CO-O-, und -O-CO-NH-, wobei der Rest $R_1$ jeweils an der rechten Seite der für $X_1$ definierten Reste gebunden ist, $Y_1$ ausgewählt wird aus der Gruppe bestehend aus -O-, $-SO_2-O-$, -CO-O, -O-CO-O- und -NH-CO-O-, wobei der Rest $R_1$ jeweils an der linken Seite der für $Y_1$ definierten Reste gebunden ist, $R_1$ einen zweiwertigen aliphatischen, cycloaliphatischen, aromatischen oder araliphatischen Rest nach dem Entfernen der funktionellen Gruppen bedeutet, der unsubstituiert ist oder der mit ein oder mehreren Alkyl-, Alkoxy-, Alkylthio-, Alkenyl-, Cycloalkyl-, Aryl-, Cyano- oder Nitroresten substituiert ist und/oder in dem gegebenenfalls ein oder mehrere Kohlenstoffatome durch Sauerstoff-, Schwefel- und/oder Stickstoffatome ersetzt sind, oder worin die Gruppe $X_1$-$R_1$ für den Fall, dass $Y_1$ -O- oder -O-CO-O- bedeutet, auch eine direkte Bindung sein kann, $R_2$ Alkyl, Alkenyl, Cycloalkyl, Aryl oder Halogen bedeutet, $R_3$ Alkyl, Cycloalkyl, Aryl oder Halogen ist, und m und n unabhängig voneinander ganze Zählen von 0 bis 4 darstellen.

Der Begriff "im wesentlichen linear" ist so zu verstehen, dass die erfindungsgemässen Polymeren entweder lineare Ketten ausbilden oder einen solchen Verzweigungsgrad aufweisen, so dass sie sich ohne Schwierigkeit in einem herkömmlichen organischen Lösungsmittel, beispielsweise in einem chlorierten Kohlenwasserstoff oder in einem polaren aprotischen Lösungsmittel, lösen lassen.

Die Löslichkeit wird im allgemeinen von der Konzentration der Struktureinheiten der Formel I beeinflusst werden. Dabei wird die Löslichkeit mit zunehmender Konzentration dieser Struktureinheiten in der Regel zunehmen. Unter "einem die Löslichkeit des Polymeren erhöhenden Anteil an Struktureinheiten der Formel I" (im Falle von Copolymeren) ist im Rahmen dieser Beschreibung zumindest ein solcher Anteil zu verstehen, der im Vergleich zu nicht mit solchen Struktureinheiten modifizierten Polymeren (Basispolymer) entweder überhaupt erst zu einer Löslichkeit in einem bestimmten Lösungsmittel führt oder der zu einer merklichen Erhöhung der Lagerstabilität einer Lösung führt. Uebliche Werte für die Konzentration einer Polymerlösung bewegen sich im Bereich von 1-75 Gew.%, insbesondere 5-50 Gew.% des Polymeren,

bezogen auf die Lösung.

Die für ein bestimmtes Polymer zum Erzielen einer gewünschten Löslichkeit in einem vorgegebenen Lösungsmittel benötigte Menge an Struktureinheiten der Formel I kann mittels Routineversuchen ermittelt werden. In der Regel enthalten die erfindungsgemässen Polymeren mehr als 5 Mol%, vorzugsweise mehr als 10 Mol%, und ganz besonders mehr als 50 Mol% dieser Struktureinheiten, bezogen auf das Polymere.

Unter dem Begriff "Polymer" sind im Rahmen dieser Beschreibung auch Oligomere zu verstehen. Die reduzierte Viskosität der erfindungsgemässen Polymeren erstreckt sich über einen Bereich von etwa 0,1 bis etwa 2,0 dl/g. Dies entspricht einem Molekulargewichtsbereich von etwa 1'000 bis etwa 100'000 (Zahlenmittel). Bevorzugt werden Polymere mit einem Zahlenmittel des Molekulargewichts von 5'000 bis 50'000. Im Falle der Diglycidylether auf Basis der erfindungsgemässen Bisphenole können die Oligomeren auch Molekulargewichte wesentlich unterhalb von 1'000 aufweisen.

Die erfindungsgemässen Polymeren weisen entweder gleiche oder unterschiedliche wiederkehrende Struktureinheiten der Formel I auf oder es handelt sich um Copolymere enthaltend neben den wiederkehrenden Struktureinheiten der Formel I noch zusätzliche wiederkehrende Struktureinheiten der Formel II,

$\{ Q \}$ (II)

worin Q sich ableitet von einem Monomeren oder einem Monomerengemisch, das sich zusammen mit den zum Aufbau der Struktureinheit der Formel I eingesetzten Monomeren copolymerisieren lässt.

Die Reste $X_1$ und $Y_1$ in den Struktureinheiten der Formel I können im Rahmen der gegebenen Definitionen gleiche oder unterschiedliche Bedeutungen aufweisen. Vorzugsweise haben diese Reste die gleiche Bedeutung.

Beispiele für Monomere, die zum Aufbau der Struktureinheiten der Formel I eingesetzt werden können, sind Dicarbonsäuren oder deren polyesterbildende Derivate, wie Dicarbonsäurehalogenide oder Ester, Disulfonsäuren oder deren polyesterbildende Derivate, wie Disulfonsäurehalogenide, Dihalogenverbindungen, wie Dichloride oder Difluoride, Diisocyanate, Diarylcarbonate, Epihalogenhydrine, Carbon-sulfonsäuren oder deren polyesterbildende Derivate, Isocyanato-sulfonsäurehaalogenide, Isocyanato carbonsäurehalogenide oder Halogencarbonyloxycarbonsäurehalogenide.

Beispiele für zusätzliche Monomere, die zum Aufbau der Struktureinheiten der Formel II eingesetzt werden können, sind neben den oben erwähnten Monomeren noch Diamine, Aminoalkohole, Dimercaptoverbindungen oder insbesondere Dihydroxyverbindungen, wie Bisphenole.

Bei den erfindungsgemässen Polymeren kann es sich um irgendwelche Polymeren handeln, sofern sie "im wesentlichen linear" im Sinne der obigen Definition sind und den oben gekennzeichneten Mindestgehalt an Strukturelementen der Formel I aufweisen.

Beispiele für erfindungsgemässe Polymere sind Polyether, Polyester, Polycarbonate, Polysulfonate, Polyurethane, Polyetheramide, Polyetherester und Polyimide. Die Polymeren der vorstehenden Aufzählung sind dabei nach den jeweils bei der Polymerisation entstehenden funktionellen Gruppen bezeichnet. Die jeweils eingesetzten Monomeren können natürlich ihrerseits funktionelle Gruppen aufweisen, die bei der Polymerisation erhalten bleiben und anschliessend im Polymergerüst auftreten. Beispiele für solche Polymere sind Polyetherketone, Polyethersulfone oder Phenoxyharze, die in der obigen Aufzählung unter Polyethern einzustufen sind oder Polyetherimide oder Polyesterimide, die in der obigen Aufzählung unter Polyimiden einzustufen sind.

Bevorzugte Struktureinheiten der Formel II sind Reste der Formel III oder IV

$\{ R_4\text{-}X_2\text{-}R_5\text{-}Y_2 \}$ (III),

$\{ R_6\text{-}X_3\text{-}R_7\text{-}Y_3 \}$ (IV),

worin $R_4$ der Rest einer Dihydroxyverbindung nach dem Entfernen der funktionellen Gruppen ist, $R_6$ der Rest eines Aminoalkohols nach dem Entfernen der funktionellen Gruppen ist, $R_5$ und $R_7$ eine der für $R_1$ definierten Bedeutungen besitzen, $Y_2$ und $Y_3$ eine der für $Y_1$ definierten Bedeutungen besitzen, $X_2$ eine der für $X_1$ definierten Bedeutungen besitzt, und $X_3$ ausgewählt wird aus der Gruppe bestehend aus $-NH-SO_2-$, -NH-CO-, -NH-CO-O- und -NH-CO-NH-.

Bedeuten irgendwelche Reste Alkyl, Alkyloxy oder Alkylthio, so handelt es sich dabei um verzweigte oder insbesondere um geradkettige Reste. Die Alkylgruppe dieser Reste weist im allgemeinen ein bis sechs Kohlenstoffatome auf. Bevorzugt werden geradkettige Alkylreste mit ein bis drei Kohlenstoffatomen, insbesondere Methylreste.

Beispiele für Alkylreste sind Methyl, Ethyl, n-Propyl, Isopropyl, n-Butyl, sek.Butyl, tert.Butyl, n-Pentyl und n-Hexyl.

Beispiele für Alkyloxyreste sind Methyloxy, Ethyloxy, n-Propyloxy, Isopropyloxy, n-Butyloxy, n-Pentyloxy und n-Hexyloxy.

Beispiele für Alkylthioreste sind Methylthio, Ethylthio, n-Propylthio, Isopropylthio, n-Butylthio, n-Pentylthio und n-Hexylthio.

Bedeuten irgendwelche Reste Alkenyl, so handelt es sich dabei um verzweigtes oder insbesondere um geradkettige Reste. Alkenylreste weisen im allgemeinen zwei bis sechs Kohlenstoffatome auf. Beispiele für Alkenylreste sind Vinyl, Prop-1-enyl, Prop-2-enyl, n-But-3-enyl, n-Pent-4-enyl oder n-Hex-5-enyl. Bevorzugt werden geradkettige Alkenylreste mit zwei oder drei Kohlenstoffatomen, insbesondere Vinyl, Prop-1-enyl oder Prop-2-enyl (Allyl). Alkenylsubstituenten werden besonders bevorzugt, da sie eine Vernetzung der erfindungsgemässen Polymeren über die Alkenylgruppen gestatten.

Bedeuten irgendwelche Reste Cycloalkyl, so handelt es sich dabei im allgemeinen um Gruppen mit fünf bis acht Ringkohlenstoffatomen. Bevorzugt wird Cyclohexyl. Beispiele für Cycloalkylreste sind Cyclopentyl, Cyclohexyl, Cycloheptyl oder Cyclooctyl.

Bedeuten irgendwelche Reste Halogen, so handelt es sich dabei im allgemeinen um Fluor, Iod oder insbesondere um Chlor oder um Brom.

Bedeuten irgendwelche Reste Aryl, so handelt es sich dabei im allgemeinen um aromatische Kohlenwasserstoffreste mit sechs bis vierzehn Kohlenstoffatomen. Bevorzugt wird Phenyl.
Beispiele für Arylreste sind Phenyl, Naphthyl, Biphenyl oder Anthryl. Die Kohlenwasserstoffreste sind in der Regel unsubstituiert. Sie können aber ihrerseits auch Substituenten tragen, wie Alkylgruppen oder Halogenatome.

Sind $R_1$, $R_5$ und/oder $R_7$ zweiwertige aliphatische Reste, so handelt es sich dabei in der Regel um Alkylengruppen mit einem bis zwölf Kohlenstoffatomen, die gegebenenfalls mit den oben angegebenen Resten substituiert sind.
Beispiele für Alkylenreste sind Methylen, Ethylen, Tri-, Tetra-, Penta-, Hexa-, Hepta-, Octa-, Nona-, Deca-, Undeca- und Dodecamethylen. Bevorzugt werden Tetra-, Hexa- und Octamethylen.
$-X_1-R_1-Y_1-$ kann vorzugsweise auch

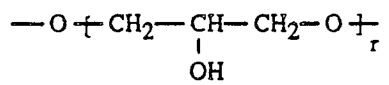

sein, worin r für eine ganze Zahl von 1 bis 5 steht, und insbesondere 1 ist.

Sind $R_1$, $R_5$ und/oder $R_7$ zweiwertige cycloaliphatische Reste, so handelt es sich dabei in der Regel um Cycloalkylengruppen mit fünf oder sechs Ringkohlenstoffatomen, die gegebenenfalls mit den oben angegebenen Resten substituiert sind und/oder die gegebenenfalls Teil einer Alkylenkette sein können. Beispiele für Cycloalkylenreste sind Cyclopentylen, Cyclohexylen, Methylcyclohexylen und 1,4-Bis-methylen-cyclohexan. Bevorzugt wird Cyclohexylen.

Sind $R_1$, $R_5$ und/oder $R_7$ zweiwertige aromatische Reste, so handelt es sich dabei in der Regel um aromatische Kohlenwasserstoffreste mit sechs bis vierzehn Ringkohlenstoffatomen, die gegebenenfalls mit den oben angegebenen Resten substituiert sind. Mehrkerninge aromatische Reste können als kondensierte Systeme vorliegen oder mehrere aromatische Systeme, wie Phenylenreste, sind über Brückenglieder, wie eine direkte C-C-Bindung oder über eine -O-, -S-,-SO-, $-SO_2-$,-CO-, $-CH_2-$, $-C(CH_3)_2-$ oder $-C(CF_3)_2$-Gruppe, miteinander verknüpft.
Beispiele für solche aromatische Kohlenwasserstoffreste sind Phenylen, Naphthylen, Biphenylen und zwei, drei oder vier über Brückenglieder miteinander verknüpfte Phenylenreste.

Bevorzugt werden 1,3- und 1,4-Phenylen sowie Reste der Formeln Va und Vb

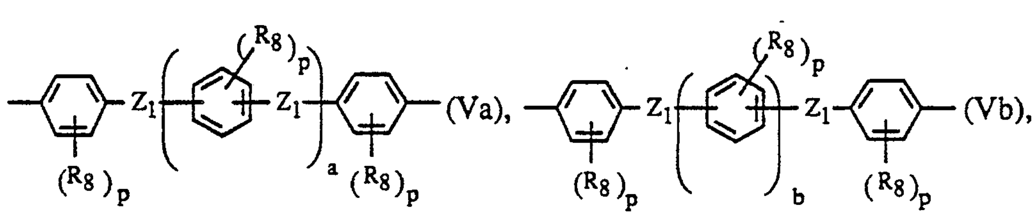

worin $R_8$ $C_1$-$C_6$Alkyl oder Halogen, insbesondere Chlor oder Brom bedeutet, p 1,2 oder insbesondere 0 ist, $Z_1$ ausgewählt wird aus der Gruppe bestehend aus -CO-, $-SO_2-$ und -SO-, a 0, 1 oder 2 bedeutetundb 2 oder 3 ist.

Sind $R_1$, $R_5$ und/oder $R_7$ zweiwertige araliphatische Reste, so handelt es sich dabei in der Regel um eine Gruppe mit sieben bis zwölf Kohlenstoffatomen, die gegebenenfalls mit den oben angegebenen Resten substituiert ist.

Ein Beispiel für einen araliphatischen Rest ist Xylylen.

Diese Reste $R_1$, $R_5$ und/oder $R_7$ können mit einem oder mit mehreren, insbesondere mit ein oder zwei Alkyl-, Alkoxy-, Alkylthio-, Alkenyl-, Cycloalkyl-, Aryl-, Cyano- oder Nitroresten substituiert sein. Beispiele für solche Substituenten sind weiter oben aufgeführt.

In diesen Resten $R_1$, $R_5$ und/oder $R_7$ können auch ein oder mehrere, vorzugsweise ein, zwei oder drei Kohlenstoffatome durch Sauerstoff-, Schwefel- und/oder Stickstoffatome ersetzt sein. Beispiele für derartig modifizierte Alkylenketten sind Reste, die sich von Polyalkylenglykolen ableiten. Ferner kann es sich um aromatische oder nicht aromatische heterocyclische Systeme handeln, die vorzugsweise fünf- oder sechsgliedrig sind. Im Falle von heterocyclischen Systemen sind vorzugsweise ein bis drei Ringkohlenstoffatome durch Stickstoffatome oder ein oder zwei Ringkohlenstoffatome durch Sauerstoff- oder Schwefelatome ersetzt. Es können auch unterschiedliche Heteroatome in einem Ring auftreten, beispielsweise ein Stickstoff- und ein Sauerstoffatom.

Beispiele für heterocyclische Reste sind Morpholin-diyl, Piperidin-diyl, Furan-diyl, Pyridin-diyl, Thiophen-diyl, Triazin-diyl und Pyridazin-diyl.

Die Definition "ein oder mehrere Kohlenstoffatome sind durch Sauerstoff-, Schwefel-und/oder Stickstoffatome ersetzt" umfasst auch solche heterocyclischen Systeme, bei denen die Ringheteroatome neben der Einbindung in das Ringsystem noch ihrerseits zusätzliche Atome oder Substituenten tragen. Beispiele für solche Heterogruppen sind $-SO_2-$,-NH- oder -NO-.

Bei dem Rest einer Dihydroxyverbindung $R_4$ und/oder dem Rest eines Aminoalkohols $R_6$ handelt es sich um einen zweiwertigen aliphatischen, cycloaliphatischen, aromatischen oder araliphatischen Rest. Beispiele dafür sind weiter oben für $R_1$ definiert. Bevorzugt handelt es sich bei $R_4$ und $R_6$ um zweiwertige aromatische Reste.

Ganz besonders bevorzugte Reste $R_4$ besitzen die Strukturen der Formeln VIa bis VIg

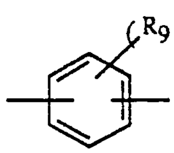 (VIa), 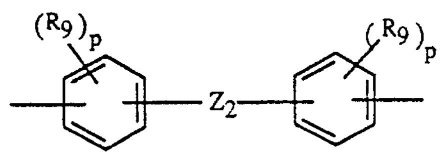 (VIb),

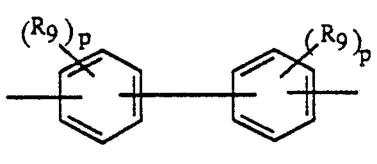 (VIc), 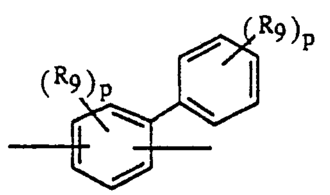 (VId),

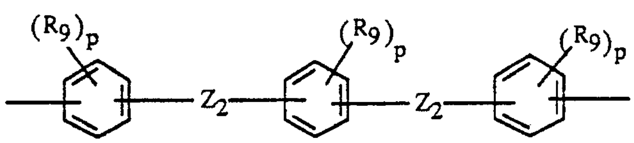 (VIe), 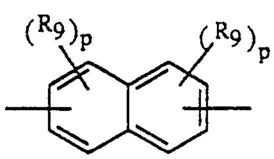 (VIf)

und 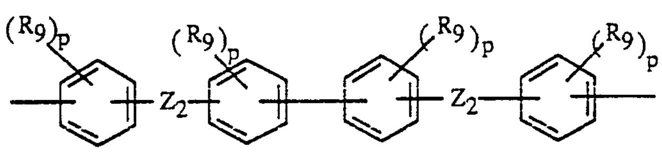 (VIg),

worin $R_9$ $C_1$-$C_6$Alkyl oder Halogen, insbesondere Chlor oder Brom bedeutet, p die oben definierte Bedeutung besitzt und $Z_2$ ausgewählt wird aus der Gruppe bestehend aus -CO-, -SO-, $-SO_2-$, -S-, -O-, $-CH_2-$, $-C(CH_3)_2-$, $-C(CF_3)_2-$, $-C(CH_3)(C_6H_5)-$ und $-C(C_6H_5)_2-$.

Ganz besonders bevorzugte Reste $R_6$ besitzen die Strukturen der oben definierten Formeln VIa, VIb und VIf, worin $Z_2$ $-CH_2-$, $-C(CH_3)_2-$ oder $-C(CF_3)_2-$ bedeutet.

Bevorzugte Polymere enthalten mindestens 5 Mol %, bezogen auf das Polymere, an Resten der Formel VII

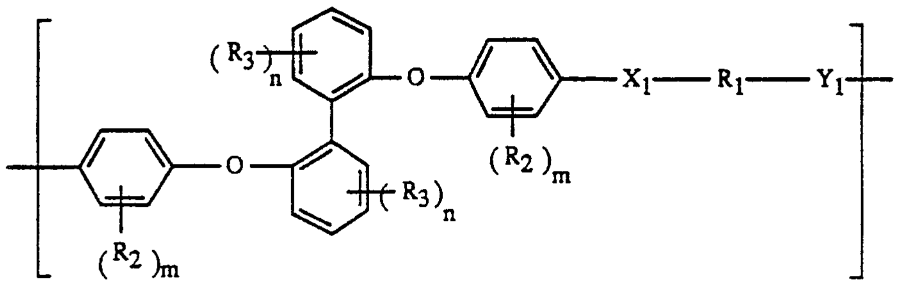

(VII),

worin $R_1$, $R_2$, $R_3$, $X_1$, $Y_1$, m und n die oben definierte Bedeutung besitzen.

Von den Polymeren dieses Typs sind besonders diejenigen bevorzugt, worin m und n 0 sind, $X_1$ -O-CO- oder insbesondere -O- ist, $Y_1$ -CO-O- oder insbesondere -O- ist, und $R_1$ ein zweiwertiger carbocyclisch-aromatischer Rest nach dem Entfernen der funktionellen Gruppen ist.

Besonders bevorzugte Polymere dieser Erfindung sind überwiegend aus aromatischen Gruppen aufgebaut. Davon sind besonders die Polyether und davon die Polyetherketone und Polyethersulfone bevorzugt. Erfindungsgemässe Polymere mit aromatischen Gruppen zeichnen sich in der Regel durch besonders gute thermische Eigenschaften aus. Dabei handelt es sich bevorzugt um Verbindungen, die zu 10 bis 100 Mol% aus wiederkehrenden Struktureinheiten der Formel I und zu 90 bis 0 Mol% aus wiederkehrenden Struktureinheiten der Formel III oder IV bestehen, worin $R_1$ und $R_4$ bis $R_7$ aromatische Reste sind.

Von den Polymeren dieses Typs sind besonders diejenigen bevorzugt, worin $X_1$ und $X_2$ ausgewählt werden aus der Gruppe bestehend aus -O-CO-, -O-CO-O- und insbesondere -O-, worin $X_3$ ausgewählt wird aus der Gruppe bestehend aus -NH-CO- und -NH-CO-NH-, und worin $Y_1$, $Y_2$, $Y_3$ ausgewählt werden aus der Gruppe bestehend aus -CO-O-, -O-CO-O- und insbesondere -O-.

Ganz besonders bevorzugt werden Polymere, die im wesentlichen aus wiederkehrenden Struktureinheiten der Formel I bestehen.

Bevorzugte Gruppen $X_1$ und $X_2$ sind -O-CO-, -O-CO-O- und insbesondere -O-.

Bevorzugte Gruppen $X_3$ sind -NH-CO- und -NH-CO-NH-.

Bevorzugte Gruppen $Y_1$, $Y_2$ und $Y_3$ sind -CO-O-, -O-CO-O- und insbesondere -O-.

Ganz besonders bevorzugte Polymere dieser Erfindung bestehen im wesentlichen aus 5 bis 100 Mol%, vorzugsweise 10 bis 100 Mol% an wiederkehrenden Struktureinheiten der Formel Ia und aus 95 bis 0 Mol %, vorzugsweise 90 bis 0 Mol% an wiederkehrenden Struktureinheiten der Formel IIa, wobei die Prozentangaben auf das Polymere bezogen sind

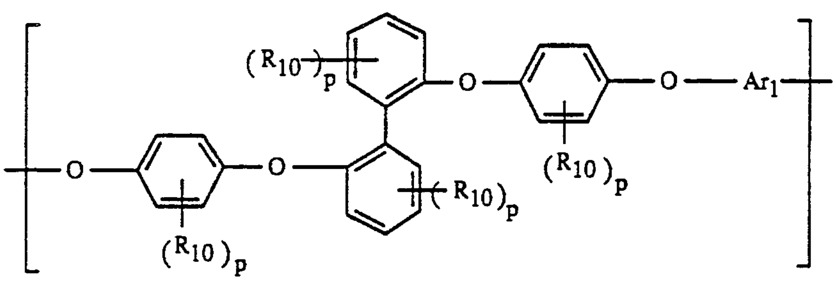

(Ia),

$-O-Ar_2-O-Ar_3-$ (IIa), worin $Ar_1$ und $Ar_3$ unabhängig voneinander zweiwertige Reste der oben definierten Formeln Va und/oder Vb sind, $Ar_2$ ein zweiwertiger Rest der oben definierten Formeln VIa, VIb, VIc, VId, VIe, VIf und/oder VIg ist, p die oben definierte Bedeutung besitzt und $R_{10}$ $C_1$-$C_3$-Alkyl, $C_3$-$C_4$-Alkenyl, Chlor oder Brom bedeutet.

Besonders bevorzugte Reste $Ar_1$ und $Ar_3$ sind

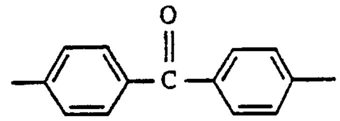 und

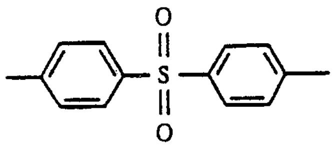 .

Besonders bevorzugte Reste $Ar_2$ sind

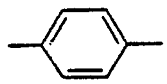 , 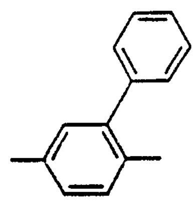 ,

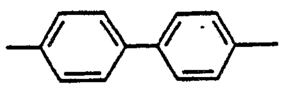 , 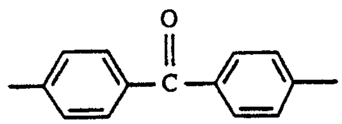 , 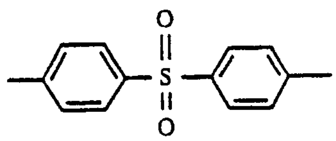 und

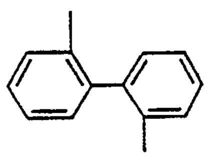 .

Weitere bevorzugte erfindungsgemässe Verbindungen, welche sich vom Bisphenol der Formel VIII ableiten

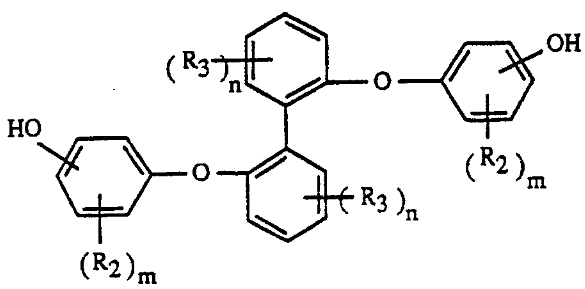

(VIII),

besitzen die allgemeine Formel IX

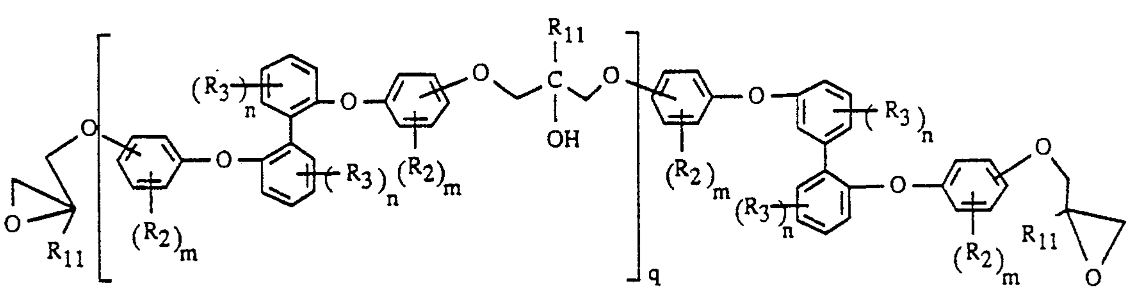

(IX),

worin $R_{11}$ $C_1$-$C_6$-Alkyl, besonders Methyl oder insbesondere Wasserstoff ist, $R_2$, $R_3$, m und n die weiter oben definierte Bedeutung besitzen und g eine ganze Zahl von 0 bis etwa 10 ist.

In dieser Ausführungsform sind die Indizes m und n vorzugsweise 0, q ist vorzugsweise eine ganze Zahl von 0 bis 5 und $R_{11}$ ist vorzugsweise Wasserstoff.

Epoxidharze enthaltend die erfindungsgemässe Bisphenoleinheit lassen sich auch erhalten, indem man beliebige Polyepoxide, insbesondere Diepoxide, mit dem Bisphenol der Formel VIII und gegebenenfalls weiteren Dialkoholen und/oder Bisphenolen vorverlängert oder indem man das Diepoxid der Formel IX, gegebenenfalls in Kombination mit weiteren Polyepoxiden oder insbesondere Diepoxiden, mit beliebigen Dialkoholen und/oder Bisphenolen vorverlängert. Setzt man die Diol- und die Epoxidkomponenten in etwa stöchiometrischen Mengen ein, so erhält man in an sich bekannter Weise Phenoxyharze, die praktisch keine Epoxidgruppen mehr aufweisen.

Diese Epoxid- und Phenoxyharze sind durch das allgemeine Strukturelement der Formel X gekennzeichnet

$-O-R_{12}-O-R_{13}-$ (X),

worin $R_{12}$ der Rest eines Dialkohols, insbesondere eines Bisphenols, nach dem Entfernen der Hydroxylgruppen ist und $R_{13}$ der hydroxylgruppenhaltige Rest einer Polyepoxidverbindung, insbesondere einer Diepoxidverbindung, ist, bei dem mindestens zwei Epoxidsauerstoffatome durch sekundäre Hydroxylgruppen ersetzt sind, mit der Massgabe, dass mindestens ein Teil der Reste $R_{12}$ eine Struktur der Formel

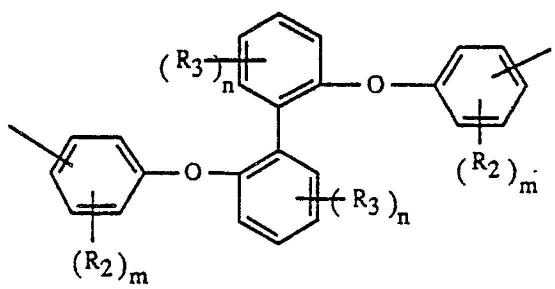

aufweist, worin $R_2$, $R_3$, m und n die oben definierte Bedeutung besitzen.

$R_{13}$ leitet sich im allgemeinen von Polyglycidylestern, Poly-(N-glycidyl)-verbindungen, Poly-(S-glycidyl)-verbindungen, cycloaliphatischen Epoxidharzen, Epoxidierungsprodukten von Dienen oder Polyenen oder insbesondere von Polyglycidylethern ab. Beispiele für solche Verbindungen sind weiter unten aufgeführt.

Eine bevorzugte Gruppe von erfindungsgemässen Epoxidverbindungen oder Phenoxyharzen weist im wesentlichen die wiederkehrende Struktureinheit der Formel XI auf

$$-O-R_{12}-O-CH_2-\underset{OH}{\underset{|}{CH}}-CH_2-X_4-R_{14}-Y_4-CH_2-\underset{OH}{\underset{|}{CH}}-CH_2- \quad \text{(XI)},$$

worin $R_{12}$ die oben definierte Bedeutung besitzt, $X_4$ -O-CO-,-NH- oder insbesondere -O-ist, wobei $R_{14}$ jeweils an der rechten Seite der für $X_4$ definierten Reste gebunden ist, $Y_4$ -CO-O-, -NH- oder insbesondere -O- ist, wobei $R_{14}$ jeweils an der linken Seite der für $Y_4$ definierten Reste gebunden ist, $X_4$ und $Y_4$ jeweils die gleiche Bedeutung besitzen, und $R_{14}$ der Rest eines Diglycidylesters, einer Di-(N-glycidyl)-verbindung, oder insbesondere eines Diglycidylethers nach dem Entfernen der Glycidylgruppen ist, mit der Massgabe, dass mindestens ein Teil der Reste $R_{12}$ eine Struktur der Formel

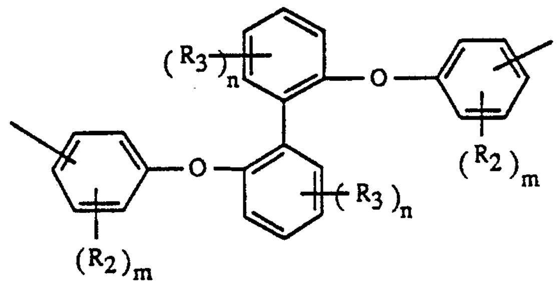

aufweist, worin $R_2$, $R_3$, m und n die oben definierte Bedeutung besitzen.

Eine besonders bevorzugte Gruppe der erfindungsgemässen Polymeren besteht im wesentlichen aus der wiederkehrenden Struktureinheit der Formel XII

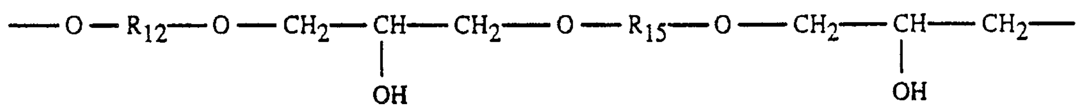

(XII),

worin $R_{12}$ die oben definierte Bedeutung besitzt und $R_{15}$ der Rest eines Diglycidylethers, vorzugsweise eines Diglycidylethers auf Basis eines Bisphenols, nach dem Entfernen der Glycidylethergruppen ist, mit der Massgabe, dass mindestens ein Teil der Reste $R_{12}$ und/oder $R_{15}$ eine Struktur der Formel

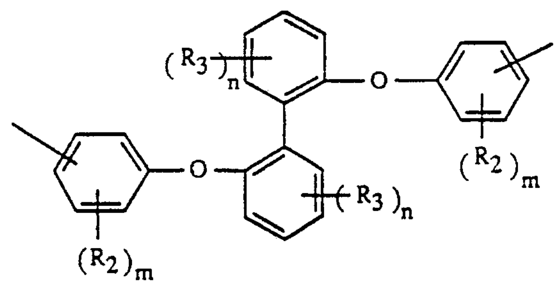

aufweist, worin $R_2$, $R_3$, m und n die oben definierte Bedeutung besitzen.

Beispiele für besonders bevorzugte Reste $R_{12}$ und $R_{15}$, die neben der oben definierten Bisphenolstruktur in den Epoxid- oder Phenoxyharzen der Formel XII auftreten können sind die oben für $R_4$ als bevorzugt definierten aromatischen Reste der Formeln VIa bis VIg.

Die erfindungsgemässen Polymeren lassen sich im allgemeinen durch Polykondensation oder Polyadditionen herstellen. Dazu setzt man eine oder mehrere Bisphenole der oben definierten Formel VIII mit einem oder mehreren geeigneten bifunktionellen Reaktionspartnern oder einem Gemisch von Reaktionspartnern um, so dass das gewünschte Polymere entsteht.

Die jeweiligen Reaktionspartner werden nach gewünschtem Endprodukt in an sich bekannter Weise ausgewählt und in an sich bekannter Weise mit dem Bisphenol der Formel VIII polykondensiert. Die Steuerung des Molekulargewichtes über die Mengenverhältnisse der einzelnen Reaktionspartner und die anzuwendenden Reaktionsbedingungen sind dem Fachmann auf dem Gebiet der Polykondensation an sich bekannt. Die einzelnen Umsetzungen können beispielsweise in Lösung, in Masse oder in Suspension erfolgen.

Bei den erfindungsgemässen Polymeren kann es sich um Homopolymere, um statistische Copolymere, Blockcopolymere oder um Pfropfcopolymere handeln. Die Endgruppen der Polymeren können die für die einzelnen Polymertypen üblichen Gruppen umfassen. Beispiele für Endgruppen sind Hydroxyl-, Alkoxyl-, Carboxyl- oder Estergruppen, Glycidylethergruppen oder Halogenatome. Weitere spezifische Endgruppen können durch Folgereaktionen hergestellt werden. Beispielsweise können Maleinimid-Endgruppen durch Reaktion eines amino-terminierten erfindungsgemässen Polymeren mit Maleinsäureanhydrid erhalten werden. Weitere Beispiele für solche Endgruppen sind Ethinyl, Benzocyclobutenyl und Nadicimidyl. Ferner können die erfindungsgemässen Polymeren in amorpher, kristalliner oder flüssig-kristalliner Form vorliegen.

Die Polykondensationen verlaufen im allgemeinen nach folgendem Schema: Das Bisphenol der Formel VIII wird in Gegenwart einer oder mehrerer mit dem Bisphenol der Formel VIII copolymerisierbarer Monomerer der Formel XIII gegebenenfalls zusammen mit weiteren Dihydroxyverbindungen der Formel XIV und/oder Aminoalkoholen der Formel umgesetzt

FG-$R_1$-FG (XIII), HO-$R_4$-OH (XIV), $H_2N$-$R_6$-OH (XV),

worin $R_1$, $R_4$ und $R_6$ die oben definierte Bedeutung besitzen, und FG eine funktionelle Gruppe ist, die zur Umsetzung mit einer Hydroxyl- oder einer primären Aminogruppe befähigt ist.

Beispiele für Gruppen FG sind polyetherbildende Reste, wie Hydroxylgruppen oder Halogenatome, insbesondere Chlor oder Fluor, polysulfonatbildende Reste, wie Sulfonsäuregruppen oder deren polyesterbildende Derivate, wie Sulfonsäurehalogenidgruppen, polyesterbildende Reste, wie Carboxylgruppen oder deren polyesterbildende Derivate, wie Estergruppen oder Säurehalogenidgruppen, polycarbonatbildende Reste, wie Halogencarbonyloxygruppen, und polyurethanbildende Reste, wie Isocyanatgruppen.

Nicht abschliessende Beispiele für mögliche Kombinationen von Reaktionspartnern für Polykondensationsreaktionen sind:

A. Kombinationen von Dialkoholen oder Bisphenolen oder deren polyetherbildenden Derivaten, wie von polyetherbildenden Chloriden oder Fluoriden, insbesondere den gegenüber einem nukleophilen Austausch aktivierten Derivaten, mit dem Bisphenol der Formel VIII und gegebenenfalls weiteren Bisphenolen.

Nukleophile Austauschreaktionen der Bisphenole oder deren Alkalisalze mit den entsprechenden aktivierten Derivaten werden in der Regel in einem inerten Lösungsmittel bei erhöhten Temperaturen und in Gegenwart eines basischen Katalysators, beispielsweise Kaliumcarbonat oder -hydroxid, vorgenommen.

Eine weitere Herstellungsmöglichkeit von erfindungsgemässen aromatischen Polyethern besteht in der Kupplung von Bisphenolen mit aromatischen Dihalogeniden. Die Reaktion wird im allgemeinen bei erhöhter Temperatur in Gegenwart eines polaren aprotischen Lösungsmittels und einem Oxidationsmittel, beispielsweise einer Kombination von Kaliumcarbonat/Kupfer-I-iodid, von $Cu_2O$ oder Cu, vorgenommen.

B. Kombinationen von Dicarbonsäuren oder deren polyesterbildenden Derivaten, wie den Säurehalogeniden oder Estern, mit dem Bisphenol der Formel VIII und gegebenenfalls weiteren Dihydroxyverbindungen und/oder Aminoalkoholen und/oder Hydroxycarbonsäuren und/oder Hydroxysulfonsäuren oder den polyesterbildenden Derivaten dieser Säuren.

Die Herstellung von Polyestern kann beispielsweise durch Umesterung von geeigneten Dicarbonsäureestern mit Bisphenolen, sowie durch Umesterung der freien Säure mit Bisphenolderivaten, wie z.B. den Diacetaten, in der Schmelze und gegebenenfalls in Gegenwart von basischen oder sauren Katalysatoren erfolgen. Geeignete Katalysatoren sind Dibutylzinnoxid, p-Toluolsulfonsäure, Bortrifluorid, Titandioxid, Calciumacetat, Manganacetat, Zinkacetat oder Antimonoxid.

Die Herstellung der Polyester kann auch durch Grenzflächenpolykondensation oder durch Polykondensation in einem inerten Lösungsmittel erfolgen. Je nach der Aktivität der eingesetzten Monomeren kann die Polykondensation bei erhöhten Temperaturen oder bei Temperaturen bis unterhalb Raumtemperatur durchgeführt werden.

C. Kombinationen von Bis-chlorcarbonylverbindungen, Diphenyl-carbonat oder Phosgen mit dem Bisphenol der Formel VIII und gegebenenfalls weiteren Dihydroxyverbindungen. Die Herstellung von Polycarbonaten erfolgt beispielsweise durch Umesterung von Diphenylcarbonat mit Bisphenolen in der Schmelze und in Gegenwart von basischen Katalysatoren, wie LiH oder NaOH.

Eine weitere Variante stellt die Umsetzung von Bisphenolen mit Phosgen oder einer Bis-chlorcarbonylverbindung in einem inerten Lösungsmittel dar. Die Umsetzung mit Phosgen oder einer Bis-chlorcarbonylverbindung kann auch durch Grenzflächenpolykondensation des Phenolats in einem Gemisch aus Wasser und einem inerten Lösungsmittel erfolgen.

D. Kombinationen von Disulfonsäuren oder deren polysulfonatbildenden Salzen oder Sulfonsäurechloriden mit dem Bisphenol der Formel VIII und gegebenenfalls weiteren Dihydroxyverbindungen und/oder Aminoalkoholen.

So kann beispielsweise die Umsetzung der Disulfonsäurechloride mit den Bisphenolen in der Schmelze der Monomeren oder in Lösung bzw. als Grenzflächenpolykondensation in einem inerten Lösungsmittel und in Gegenwart einer Base, beispielsweise von Kaliumcarbonat oder -hydroxid, erfolgen.

E. Kombinationen von Dianhydriden enthaltend einen vom Bisphenol der Formel VIII abgeleiteten Rest mit Diaminen

Die Umsetzung dieser Monomeren kann in einem inerten Lösungsmittel vorzugsweise bei Temperaturen unterhalb von 50°C erfolgen. Dabei entsteht im allgemeinen zunächst eine Polyamidsäure, die anschliessend in an sich bekannter Weise thermisch oder chemisch, beispielsweise mit Essigsäureanhydrid,

gegebenenfalls unter der Katalyse eines tertiären Amins, wie beispielsweise Triethylamin oder Pyridin, zum Polyimid cyclisiert wird.

Bei den in den oben beschriebenen Reaktionen eingesetzten inerten Lösungsmitteln kann es sich um irgendwelche organische Lösungsmittel handeln, die in der Lage sind, mindestens einen, vorzugsweise alle Reaktionspartner zu lösen und die unter den Reaktionsbedingungen im wesentlichen inert sind. Beispiele dafür sind aromatische Kohlenwasserstoffe, wie Toluol oder Xylol, chlorierte aliphatische Kohlenwasserstoffe, wie Methylenchlorid, Chloroform oder 1,1,2-Trichlorethan, oder polare aprotische Lösungsmittel, wie Dimethylsulfoxid, N-Methylpyrrolidon, N,N-Dimethylformamid, N,N-Dimethylacetamid und Diphenylsulfon.

Werden die oben beschriebenen Reaktionen unter erhöhten Temperaturen durchgeführt, so handelt es dabei um Temperaturen, bei denen die jeweilige Reaktion mit der jeweils gewunschten Geschwindigkeit abläuft. Typische Temperaturen bei diesen Umsetzungen bewegen sich im Bereich von etwa 100 °C bis zum Siedepunkt des jeweiligen Lösungsmittels. Wird eine Umsetzung in der Schmelze der Monomeren durchgeführt, so können Temperaturen bis oberhalb von 300°C angewendet werden.

Beispiele für geeignete Dialkohole sind:
Ethylenglykol, Diethylenglykol, Propan-1,2-diol, Propan-1,3-diol, Butan-1,4-diol, Poly-(oxyethylenglykol), Poly-(oxypropylenglykol), Poly-(oxybutylenglykol), Pentan-1,5-diol, Hexan-1,6-diol, Octan-1,8-diol,2,2-Dimethylpropan-1,3-diol, trans-Tetramethylcyclobutandiol und 1,4-Bis-(hydroxymethyl)-cyclohexan.

Beispiele für geeignete Bisphenole sind:
Hydrochinon, Chlor-, Methyl-, 2,3-Dimethyl- oder Trimethylhydrochinon, Tetrafluorhydrochinon, Resorcin, 1,2-Bis-(4-hydroxyphenyl)-ethan, 2,2-Bis-(4-hydroxyphenyl)propan (Bisphenol A), Tetrabrombisphenol A, Hexafluorbisphenol A, Bis-(4-hydroxyphenyl)-ether, Bis-(4-hydroxyphenyl)-sulfid, Bis-(4-hydroxyphenyl)-sulfon, Bis-(hydroxyphenyl)-methan (Bisphenol F), Bis-(4-hydroxy-3,5-dimethylphenyl)-methan, 2,2-Bis-(4-hydroxy-3-methylphenyl)-propan, 2,2-Bis-(4-hydroxy-3,5-dimethylphenyl)-propan, 2,2-Bis-(4-hydroxy-3,5-dichlorphenyl)-propan, Bis-(4-hydroxy-3,5-dimethylphenyl)-sulfid, Bis-(4-hydroxy-3,5-dimethylphenyl)-sulfon, 2,2-Bis-(4-hydroxy-3,5-dibromphenyl)-propan, 4,4′-Dihydroxybiphenyl, 4,4′-Dihydroxy-2,2′-dimethylbiphenyl, 2,2′-Dihydroxybiphenyl, 2,5-Dihydroxybiphenyl, 4,4′-Dihydroxyterphenyl, 4,4′-Dihydroxybenzophenon, 2,2′-Dihydroxybenzophenon, 4,4′-Dihydroxydiazodiphenyl, 4,4′-Dihydroxyterephthalophenon, 4,4′-Dihydroxyisophthalophenon, 4,4′-Dihydroxytriphenylmethan, Phenolphthalein, Phenolsulfonphthalein, Fluorescein, 9,9-Bis-(4-hydroxyphenyl)-fluoren, 1,5-Dihydroxyanthrachinon, 2,6-Dihydroxyanthrachinon, 1,5-Dihydroxynaphthalin, 1,6-Dihydroxynaphthalin, 2,6-Dihydroxynaphthalin, 2,7-Di-hydroxynaphthalin, 4,5-Bis-(4-hydroxyphenyl)-2-phenylimidazol und 2,3-Bis-(4-hydroxyphenyl)-chinoxalin.

Beispiele für geeignete polyetherbildende Halogenverbindungen sind:
1,3-Dibrombenzol, 1,4-Dibrombenzol, 4,4′-Dibrombiphenyl, 4,4′-Dibrombenzophenon, 2,7-Dibrom-9-fluorenon, Bis-(1,3-chlormethyl)-benzol, Bis-(1,4-chlormethyl)-benzol, Bis-(1,4-brommethyl)-benzol, Bis-(1,5-chlormethyl)-naphthalin, Bis-(4-chlorphenyl)-sulfon, Bis-(4-fluorphenyl)-sulfon, Bis-(4-chlorphenyl)-keton, Bis-(4-fluorphenyl)-keton, 4,6-Di-chlor-2-phenyl-1,3,5-triazin, 4,6-Di-chlor-2-methyl-1,3,5-triazin, 4,6-Di-chlor-2-methoxy-1,3,5-triazin oder 3,6-Dichlorpyridazin.

Beispiele für geeignete Dicarbonsäuren oder deren poly-esterbildende Derivate sind:
Adipinsäure, Azelainsäure, Sebacinsäure, Süberinsäure, Pimelinsäure, Hexåhydroterephthalsäure, Terephthalsäure, Isophthalsäure, 2,5-Dichlorterephthalsäure, 2,5-Dichlorisophthalsäure, 2-Phenylisophthalsäure, 5-Methylisophthalsäure, 4,4′-Biphenyldicarbonsäure, Diphenylsulfon-4,4′-dicarbonsäure, Diphenylether-4,4′-dicarbonsäure, Diphenylether-3,4′-dicarbonsäure, Diazodiphenyl-4,4′-dicarbonsäure, Diphenylmethan-4,4′-dicarbonsäure, 2,2-Bis(4-benzoesäure)-propan, 2,2-Bis-(4-benzoesäure)-hexafluoropropan, Triphenylphosphinoxid-4,4′-dicarbonsäure, Diphenylmethylphosphinoxid-4,4′-dicarbonsäure, Naphthalin-2,6-dicarbonsäure, 1,4-Cyclohexandicarbonsäure, [2.2.2]-Bicyclooctandicarbonsäure und [3.2.2]-Bicyclononandicarbonsäure, sowie die Methyl-, Ethyl- oder Phenylester dieser Säuren oder deren Säurechloride.

Beispiele für geeignete Hydroxycarbonsäuren oder deren polyesterbildende Derivate sind:
4-Hydroxybenzoesäure, 3-Chlor-4-hydroxybenzoesäure, 3,5-Dichlor-4-hydroxybenzoesäure, 3-Methyl-4-hydroxybenzoesäure, 3-Brom-4-hydroxybenzoesäure, 3,5-Dimethyl-4-hydroxybenzoesäure, 3-Methoxy-4-hydroxybenzoesäure, 6-Hydroxy-2-naphthoesäure, 6-Hydroxy-5-chlor-2-naphthoesäure und 6-Hydroxy-5-methyl-2-naphthoesäure sowie die Methyl-, Ethyl- oder Phenylester dieser Säuren oder deren Säurechloride, sowie die Monoester dieser Phenole, wie die Acetate.

Beispiele für geeignete Hydroxysulfonsäuren oder deren polyesterbildende Derivate sind die Analogen der oben aufgezählten Hydroxycarbonsäure(derivate), bei denen die Carboxylgruppe durch eine Sulfonsäuregruppe ersetzt ist.

Beispiele für geeignete Aminophenole sind:
3-oder 4-Aminophenol, 2-Hydroxy-5-amino-naphthalin, 2-Hydroxy-6-amino-naphthalin oder 2-Hydroxy-7-amino-naphthalin.

Beispiele für geeignete Disulfonsäure(derivate) sind:
1,3-Benzoldisulfonsäure, 1,4-Benzoldisulfonsäure, 4,4′-Biphenyldisulfonsäure, 4,4′-Oxy-bis-(benzolsulfonsäure), 4,4′-Sulfonyl-bis-(benzolsulfonsäure), 4,4′-Methylen-bis-(benzolsulfonsäure) und 2,2-Bis-(benzolsulfonsäure)-propan und die Disulfonsäurechloride bzw. die Dinatriumsalze dieser Säuren.

Ein Beispiel für ein geeignetes Isocyanatosulfonsäurehalogenid ist 4-Isocyanatophenylsulfonsäurechlorid.

Ein Beispiel für ein geeignetes Isocyanatocarbonsäurehalogenid ist 4-Isocyanatophenylcarbonsäurechlorid.

Ein Beispiel für ein geeignetes Halogencarbonyloxycarbonsäurehalogenid ist 4-Chlorcarbonyloxyphenylcarbonsäurechlorid.

Beispiele für Dianhydride enthaltend einen vom Bisphenol der Formel VIII abgeleiteten Rest sind Umsetzungsprodukte eines Bisphenols der Formel VIII mit zwei Anteilen eines eine nukleophil austauschbare Gruppe aufweisenden Anhydrids einer aromatischen Dicarbonsäure, beispielsweise eines 3- oder 4-Nitro-, -Chlor- oder -Bromphthalsäureanhydrids. Beispiele für Dianhydride enthaltend einen vom Bisphenol der Formel VIII abgeleiteten Rest sind Umsetzungsprodukte eines Bisphenols der Formel VIII mit zwei Anteilen eines Anhydrids einer aromatischen Tricarbonsäure, beispielsweise Trimellitsäureanhydrid. Diese Dianhydride weisen die allgemeine Formel XVI auf

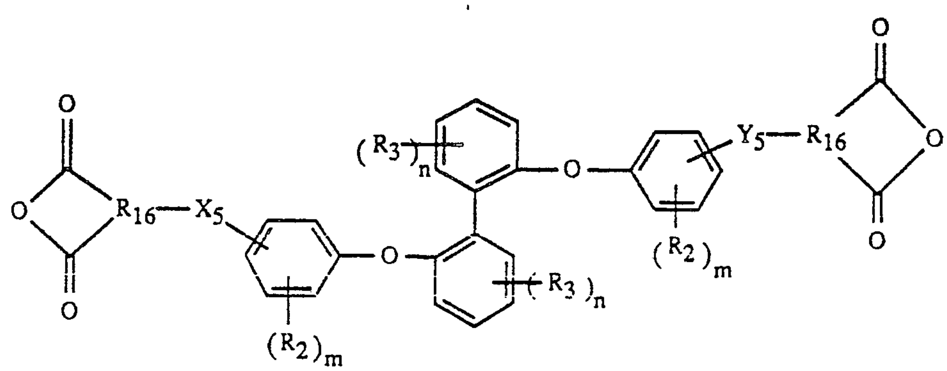

(XVI),

worin $R_2$, $R_3$, m und n die oben definierte Bedeutung besitzen, $X_5$ -O- oder -O-CO- ist, $Y_5$ -O- oder -CO-O- ist und $R_{16}$ ein dreiwertiger aromatischer Rest ist, wobei der Rest $R_{16}$ jeweils an der linken Seite der für $X_5$ definierten Reste und jeweils an der rechten Seite der für $Y_5$ definierten Reste gebunden ist. Diese Dianhydride lassen sich beispielsweise in Kombination mit Diaminen zur Herstellung von Polyether- oder Polyesterimiden verwenden.

Beispiele für geeignete Diamine sind:
1,6-Diaminohexan, 1,4-Diaminocyclohexan, Bis-(4-aminocyclohexyl)-methan, 1,3-Bis-(aminomethyl)-cyclohexan, 1,4-Bis-(aminomethyl)-cyclohexan, m-Phenylendiamin, p-Phenylendiamin, 2,4-Diaminotoluol, 1,4-Diamino-2,5-dichlorbenzol, 4,4′-Diaminodiphenylether, 3,4′-Diaminodiphenylether, 4,4′-Diaminodiphenylmethan, 4,4′-Diaminodiphenylsulfon, 3,3′-Diaminodiphenylsulfon, 3,3′-Diaminobenzophenon, 1,5-Diaminonaphthalin, Bis-(4-amino-3-methyl-phenyl)-methan, Bis-(4-amino-3,5-diethylphenyl)-methan und 2,2′-Bis-(4-aminophenoxy)-biphenyl, 2,5-Bis-(4-aminophenyl)-oxadiazol und 2,2′-Bis-(aminophenyl)-5,5′-dibenzimidazol.

Die Herstellung der erfindungsgemässen Diglycidylether kann in an sich bekannter Weise durch Umsetzung des Bisphenols der Formel VIII mit Halogenhydrinen erfolgen. So lässt sich beispielsweise eine Verbindung der Formel VIII mit Epichlorhydrin, Glycerindichlorhydrin oder mit β-Methylepichlorhydrin unter alkalischen Bedingungen oder in Anwesenheit eines sauren Katalysators mit anschliessender Alkalibehandlung umsetzen.

Ein Teil der erfindungsgemässen Polymeren kann auch über eine Polyadditionsreaktion hergestellt werden. Ein Beispiel hierfür ist die an sich bekannte Vorverlängerung des erfindungsgemässen Diglcidylethers mit einem Unterschuss an weiteren Dihydroxyverbindungen, insbesondere mit dem Bisphenol der Formel VIII oder mit anderen Bisphenolen, wodurch Diglcidylether höheren Molekulargewichts entstehen. Man kann aber auch beliebige Polyepoxide, insbesondere Diepoxide, mit einem Unterschuss des Bisphenols der Formel VIII und gegebenenfalls weiteren Dialkoholen und/oder Bisphenolen vorverlängern.

Ein weiteres Beispiel für eine Polyadditionsreaktion ist die Herstellung von Phenoxyharzen durch Umsetzung etwa stöchiometrischer Mengen von Polyglycidylverbindungen, insbesondere von Diglycidylethern auf Basis von Bisphenolen, wie Bisphenol F oder insbesondere Bisphenol A, mit dem Bisphenol der Formel VIII gegebenenfalls in Kombination mit anderen Bisphenolen oder Dialkoholen in Gegenwart von

Basen, wie Natrium- oder Kaliumhydroxid. Anstelle des Bisphenols der Formel VIII kann man auch den Diglyidylether auf Basis dieses Bisphenols einsetzen und diesen mit etwa der stöchiometrischen Menge eines anderen Bisphenols oder des Bisphenols der Formel VIII umsetzen.

Bespiele für Polyglycidylverbindungen, die in diesen Umsetzungen eingesetzt werden können, sind Diglycidylester der oben aufgezählten Dicarbonsäuren, Poly-(N-glycidyl)-verbindungen der oben aufgezählten Diamine, Poly-(S-glycidyl)-verbindungen, wie Di-S-glycidylverbindungen von Ethan-1,2-dithiol oder von Bis-(-4-mercaptomethylphenyl)-ether, cycloaliphatische Epoxidharze und Epoxidierungsprodukte von Dienen oder Polyenen, die beispielsweise durch Epoxidierung ethylenisch ungesättigter Verbindungen hergestellt werden können, oder insbesondere Diglycidylether der oben aufgezählten Dialkohole und Bisphenole.

Ein weiteres Beispiel für die Herstellung erfindungsgemässer Polymerer über eine Polyaddition ist die Umsetzung von Diisocyanaten mit dem Bisphenol der Formel VIII gegebenenfalls in Kombination mit anderen Dihydroxyverbindungen, Aminoalkoholen oder Diaminen zu Polyurethanen oder Polyharnstoffen.

Beispiele für geeignete Dihydroxyverbindungen, Aminoalkohole oder Diamine für diese Umsetzungen sind weiter oben als Polykondensationskomponenten aufgeführt.

Beispiele für geeignete Polyisocyanate sind:
1,4-Cyclohexandiisocyanat, Isophorondiisocyanat, 4,4′-Bis-(isocyanatocyclohexyl)-methan, m-Phenylen-diisocyanat, p-Phenylen-diisocyanat, Toluol-2,4-diisocyanat, Naphthalin-1,5-diisocyanat, 4,4′-Diphenylmethandiisocyanat, m-Xylylendiisocyanat und 4,4′-Bis-(isocyanato)-3,3′-dimethylbiphenyl.

Die Herstellung der besonders bevorzugten Polyetherketone oder Polyethersulfone erfolgt beispielsweise durch Umsetzung von etwa stöchiometrischen Mengen Bisphenol und einem aromatischen Difluoro- oder Dichloroketon bzw. -sulfon. Neben dem Bisphenol der Formel VIII können selbstverständlich auch Gemische mit anderen Bisphenolen verwendet werden. Als aromatisches Difluoro- oder Dichloroketon bzw. -sulfon können bespielsweise Verbindungen der Formel XVII oder XVIII oder Gemische davon eingesetzt werden

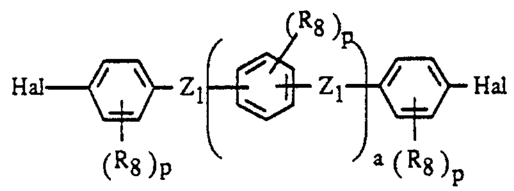
(XVII),

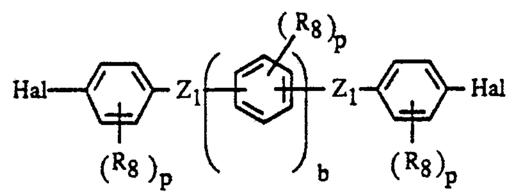
(XVIII),

worin Hal Chlor oder Fluor bedeutet und $Z_1$, $R_8$, a, b und p die oben definierte Bedeutung besitzen.

Weitere Einzelheiten betreffend die Herstellung von Polyarylethern durch nukleophile Substitution sind beispielsweise in dem Artikel von R.N. Johnson et al. in J. Polymer Sci., Part A-1, Vol 5,2375-2427(1967) oder in der EP-A-1879 zu finden.

Die Zwischenprodukte der Formel VIII sind neu und ebenfalls ein Gegenstand der vorliegenden Erfindung.

Diese Verbindungen lassen sich ausgehend von dem kommerziell erhältlichen 2,2′-Dihydroxybiphenyl herstellen. Dazu setzt man diese Verbindung mit etwa der doppelt stöchiometrischen Menge eines Halogenacetophenons um, beispielsweise mit 2-, 3- oder 4-Fluoracetophenon. Das erhaltene Bis-acetylphenoxybiphenyl wird anschliessend durch Baeyer-Villiger Oxidation in das entsprechende Bis-acetoxyphenoxybiphenyl übergeführt und in einer Folgeoperation zum Produkt der Formel VIII verseift.

Die Umsetzung von 2,2′-Dihydroxybiphenyl mit dem Halogenacetophenon erfolgt vorzugsweise in einem Lösungsmittel für beide Ausgangsprodukte, beispielsweise in einem polaren aprotischen Lösungsmittel wie N-Methylpyrrolidon, und in Gegenwart einer organischen oder insbesondere einer anorganischen Base, wie Alkalimetallcarbonat oder -hydroxid, um den entstehenden Halogenwasserstoff zu neutralisieren. Die Umsetzung erfolgt im allgemeinen bei erhöhter Temperatur, beispielsweise unter Rückfluss des jeweiligen Lösungsmittels.

Die Baeyer-Villiger Oxidation des Bis-acetylphenoxybiphenyls wird im allgemeinen unter Bedingungen durchgeführt, wie sie beispielsweise in Org. React., 9 73-107(1957) beschrieben sind. Die Reaktion kann

mit Persäuren in gegenüber den Reaktanden inerten Lösungsmitteln, wie Methylenchlorid, Chloroform, Dioxan, Acetonitril, Toluol oder Eisessig, gegebenenfalls in Gegenwart starker anorganischer oder organischer Säuren, wie Schwefelsäure, Perchlorsäure, p-Toluolsulfonsäure, Methansulfonsäure oder Trifluoressigsäure, bei Temperaturen im Bereich von etwa -20°C bis etwa 50°C durchgeführt werden. Als Oxidationsmittel sind anorganische und/oder organische Perverbindungen, wie beispielsweise Perschwefelsäure, Permonophosphorsäure, Peressigsäure, Chlorperbenzoesäure oder Wasserstoffperoxid an sich bekannt und brauchbar.

Die Verseifung des Bis-acetoxyphenoxybiphenyls kann in an sich bekannter Weise durch Erhitzen in alkalischem Medium durchgeführt werden. Dazu wird das Ausgangsmaterial in der Regel in einer wässrigen Lösung, beispielsweise in einem Gemisch von Ethanol und Wasser, in Gegenwart einer Base, beispielsweise eines Alkalimetallhydroxids, solange unter Rückfluss des Lösungsmittels gekocht, bis die Acetylgruppe abgespalten ist.

Die Isolierung und Aufarbeitung der Reaktionsprodukte der einzelnen Stufen kann mittels Routineverfahren erfolgen.

Die thermoplastischen erfindungsgemässen Polymeren, insbesondere die aromatischen Polyether, Polyetherketone oder Polyethersulfone, können in der für Thermoplaste üblichen Weise eingesetzt werden und z.B. zu Formkörpern oder Folien verarbeitet werden, oder als Matrixharze, Klebstoffe oder Ueberzugsmittel eingesetzt werden.

Die erfindungsgemässen Diglycidylether können in der für Epoxidharze üblichen Weise eingesetzt werden. Sie eignen sich vor allem in Kombination mit an sich üblichen Härtern und gegebenenfalls Härtungsbeschleunigern zur Herstellung von vernetzten Produkten. Gegebenenfalls setzt man auch Gemische der erfindungsgemässen Diglycidylether mit anderen an sich üblichen Epoxidharzen ein.

Beispiele für Epoxidhärter sind Polyamine mit mindestens zwei primären und/oder sekundären Aminogruppen, Amide einschliesslich der substituierten Harnstoffe, Polyaminoamide, Polyphenole, Polythiole, Polycarbonsäuren und insbesondere deren Anhydride, katalytisch wirkende Härtungsmittel, wie beispielsweise tertiäre Amine, Imidazole oder Mannichbasen, Zinnsalze von Alkansäuren, Friedel-Crafts-Katalysatoren und deren Komplexe und Chelate, oder Amidine, wie beispielsweise Dicyandiamid.

Beispiele für Härtungsbeschleuniger sind tertiäre Amine, deren Salze oder quaternäre Ammoniumverbindungen oder substituierte Harnstoffe. Diese Härter und Hartungsbeschleuniger sind dem Fachmann auf dem Gebiet der Epoxidverarbeitung an sich bekannt und beispielsweise im "Epoxy Handbook" von Lee and Neville beschrieben.

Vor der Verarbeitung der beispielsweise als Presspulver, Schmelze oder insbesondere als Lösung vorliegenden Polymeren können übliche Zusatzstoffe, wie beispielsweise Füllstoffe, Pigmente, Stabilisatoren oder Verstärkungsmittel, wie Kohlenstoff-, Bor-, Metall- oder Glasfasern, zugegeben werden. Die erfindungsgemässen Polymeren können auch zusammen mit anderen Thermoplasten oder Duromeren verarbeitet werden.

Vorzugsweise eignen sich die erfindungsgemässen Polymeren, insbesondere die aromatischen Polyether, Polyetherketone oder Polyethersulfone, als Matrixharze für die Herstellung von Faserverbundsystemen, wobei man als Verstärkungsfasern die üblichen bei der Verstärkung technischer Werkstoffe verwendeten Fasern einsetzen kann. Diese können organische oder anorganische Fasern, Naturfasern oder Synthesefasern sein, und als Faserbündel, als orientierte oder nicht-orientierte Fasern oder als Endlosfasern vorliegen.

Eine weitere bevorzugte Anwendungsmöglichkeit für die erfindungsgemässen Polymeren, insbesondere für die aromatischen Polyether, Polyetherketone oder Polyethersulfone, besteht in der Modifizierung anderer Kunststoffe. Diese können grundsätzlich Thermoplaste oder Duromere sein. Besondere Bedeutung erlangen solche Systeme als Matrixharze, welche zur Herstellung von Verbundbauteilen zum Einsatz gelangen.

Besonders hervorzuheben ist die unerwartet hohe Löslichkeit der erfindungsgemässen Polymeren in einer Reihe von herkömmlichen organischen Lösungsmitteln, wie in fluorierten oder chlorierten Kohlenwasserstoffen, und die sehr gute Stabilität dieser Lösungen. Beispiele für geeignete Lösungsmittel sind polare aprotische Lösungsmittel, wie N-Methylpyrrolidon, N,N-Dimethylformamid, Dimethylsulfoxid und Sulfolan, fluorierte oder chlorierte aliphatische und aromatische Kohlenwasserstoffe, wie Methylenchlorid, Tri-oder Tetra-chlorethan, Fluor-dichlorethan oder Chlorbenzol, oder cyclische Ether, wie Tetrahydrofuran oder Dioxan, sowie auch Cyclohexanon.

Die Erfindung betrifft daher auch eine Lösung enthaltend etwa 1 bis 75 Gew.%, vorzugsweise 5 bis 50 Gew.%, bezogen auf die Lösung, eines erfindungsgemässen Polymers, vorzugsweise eines erfindungsgemässen aromatischen Polymers, insbesondere eines erfindungsgemässen Polyarylenetherketons oder Polyarylenethersulfons gelöst in einem organischen Lösungsmittel.

Die folgenden Beispiele erläutern die Erfindung.

## I. Herstellung der Zwischenprodukte

### I.1. 2,2'-Bis-(4-hydroxyphenoxy)-biphenyl

#### I.1.1. 2,2'-Bis-(4-acetylphenoxy)-biphenyl

Zu einer am Wasserabscheider unter Rückfluss kochenden Suspension von 466 g (2,5 Mol) 2,2'-Dihydroxybiphenyl und 778 g (5,62 Mol) $K_2CO_3$ in 1,5 l 1-Methyl-2-pyrrolidon (NMP) und 900 ml Xylol, werden bei 145 °C 606 ml 4-Fluoracetophenon zugetropft. Nach beendeter Zugabe wird bis zum Verschwinden der Edukte am Wasserabscheider unter Rückfluss erhitzt (20-40 Std.).
Anschliessend wird das Xylol aus dem Reaktionsgefäss abdestilliert und die verbleibende Suspension nach Abkühlen auf Raumtemperatur auf 15 l Wasser gegossen. Der Niederschlag wird abfiltriert, ein zweites Mal in 5 l Wasser kräftig gemixt, filtriert und mit Wasser gewaschen.
Das Rohprodukt wird im Vakuumtrockenschrank bei 80 °C getrocknet. Eine Umkristallisation aus Toluol ergibt 653 g 2,2'-Bis-(4-acetylphenoxy)-biphenyl vom Schmp. 153-154 °C.

#### I.1.2. 2,2'-Bis-(4-acetoxyphenoxy)-biphenyl

Zu einer Suspension von 650 g (1,54 Mol) 2,2'-Bis-(4-acetylphenoxy)-biphenyl in 2,4 l Eisessig werden bei Raumtemperatur und unter $N_2$ eine Lösung von 5,7 g p-Toluolsulfonsäure in 1540 ml 40 %-iger Peressigsäure zugetropft. Dabei steigt die Temperatur auf 35 °C. Es wird 20 Std. bei Raumtemperatur gerührt und die Reaktionslösung wird dann unter kräftigem Mixen auf 20 l Wasser gegossen. Der Niederschlag wird abfiltriert und ein zweites Mal in 10 l Wasser gemixt. Restliches Peroxid wird durch Zugabe von Natriumsulfit zerstört. Das Produkt wird abfiltriert, mit Wasser gewaschen und ohne zu trocknen dem nächsten Syntheseschritt unterworfen.

#### I.1.3. 2,2'-Bis-(4-hydroxyphenoxy)-biphenyl

Eine Lösung von 1,5 Mol 2,2'-Bis-(4-acetoxyphenoxy)-biphenyl und 690 g (12,3 Mol) KOH in 1,2 l Ethanol und 4 l Wasser wird 2 Std. unter Rückfluss erhitzt. Nach Abkühlen auf Raumtemperatur wird die Lösung unter Mixen auf 20 l 10 HCl/Eis gegossen, der Niederschlag abfiltriert und mit Wasser neutral gewaschen. Das Rohprodukt wird im Vakuumtrockenschrank bei 80 °C getrocknet.
Zur Reinigung werden 420 g Rohprodukt in 4 l Chloroform unter Rückfluss erhitzt. Nach Abkühlen auf Raumtemperatur wird der Niederschlag abfiltriert, mit 1 l Chloroform gewaschen und im Vakuumtrockenschrank getrocknet. Ausbeute: 315 g 2,2'-Bis(4-hydroxyphenoxy)-biphenyl vom Schmp. 174-175 °C.
100 g des auf diese Weise gereinigten Produktes werden in 300 ml Tetrahydrofuran (THF) bei Raumtemperatur gelöst und unter Rühren 900 ml Hexan zugegeben. Dabei bildet sich ein geringer brauner Rückstand. Die überstehende klare Lösung wird auf 4 l Hexan gegossen und der sich bildende Niederschlag abfiltriert, mit Hexan gewaschen und im Vakuumtrockenschrank bei 80 °C getrocknet.
Ausbeute: 80 g 2,2'-Bis-(4-hydroxyphenoxy)-biphenyl vom Schmp. 174,5- 175,2 °C.

| Elementaranalyse berechnet für $C_{24}H_{18}O_4$: | | |
|---|---|---|
| Ber. | = C: 77,82; | H: 4,90 |
| Gef. | = C: 77,77; | H: 4,87. |

## II. Beispiele für Polymere mit 2,2'-Bis-(4-hydroxyphenoxy)-biphenyl

### II.1. Polyetherketon

In einem Rundkolben mit Rührer und Schutzgasanschluss wird unter Stickstoff eine Mischung aus 32,70 g (0,0884 mol) 2,2′-Bis-(4-hydroxyphenoxy)-biphenyl, 80,30 g Diphenylsulfon, 14,16 g (0,1025 mol) Kaliumcarbonat und 64 g Xylol bei einer Badtemperatur von 200 °C erhitzt und ein Xylol/Wasser-Gemisch abdestilliert. Gegen Ende des Destillationsvorganges wird dabei kurzzeitig Vakuum (2 mbar) angelegt. Sodann werden 19,29 g (0,0883 mol) 4,4′-Difluorbenzophenon zu der Reaktionsmischung gegeben, die Temperatur innerhalb von 25 Minuten auf 250 °C erhöht und dort 1 Stunde belassen. Danach wird die Temperatur auf 275 °C (30 Minuten) und anschliessend auf 300 °C erhöht. Diese Temperatur wird 4 Stunden beibehalten, wobei die Reaktionsmischung in zunehmendem Masse viskos wird.
Nach Abkühlen wird das Reaktionsgemisch dem Kolben entnommen, pulverisiert, mit verdünnter Essigsäure versetzt und zunächst mit Wasser und dann mit Aceton extrahiert. Anschliessend wird das Polymere in Methylenchlorid gelöst, eine geringe Menge unlösliches Material abfiltriert und in Isopropanol ausgefällt. Das so gereinigte Polymere wird danach im Vakuumtrockenschrank bis zu einer Temperatur von 240 °C getrocknet. Ein auf diese Weise hergestelltes Polyarylenetherketon besitzt eine reduzierte Viskosität (1 Gew.-% Polymeres in NMP bei 25 °C) von 0,27 dl/g. Die durch DSC bestimmte Glasübergangstemperatur liegt bei 141 °C. Das IR-Spektrum zeigt für Polyetherketone typische Banden [$^{\nu}C=O$ = 1653 $cm^{-1}$ (in KBr)]. Es ist zu mehr als 25 Gew.-% in Methylenchlorid löslich.

II.2. Polyethersulfon

In zu Beispiel II.1. analoger Weise wird ein Polyethersulfon aus 2,2′-Bis-(4-hydroxyphenoxy)-biphenyl (0,0501 mol) und 4,4′-Dichlordiphenylsulfon (0,0502 mol) mit Kaliumcarbonat (0,0556 mol) hergestellt (Reaktionsbedingungen: 1h/250 °C, 1h/275 °C, 3 h/300 °C). Das resultierende Polymere (red. Visk. = 0,23 dl/g) besitzt eine Glasübergangstemperatur von 162 °C. Es ist zu mehr als 25 Gew.-% in Methylenchlorid löslich. Diese Lösung ist über mehr als 100 Stunden stabil, d.h. es erfolgt kein Ausfallen des Polymeren.

II.3. Polyethersulfon - Copolymeres

In zu Beispiel II.1. analoger Weise wird ein Polyethersulfon aus 2,2′-Bis-(4-hydroxyphenoxy)-biphenyl (0,0250 mol), 4,4′-Dihydroxydiphenylsulfon (0,0751 mol) und 4,4′-Dichlordiphenylsulfon (0,1002 mol) mit Kaliumcarbonat (0,1103 mol) hergestellt (Reaktionsbedingungen: 1h/250 °C, 5h/320 °C). Das resultierende Polymere (red. Visk. = 0,29 dl/g) besitzt eine Glasübergangstemperatur von 203 °C. Es ist zu mehr als 25 Gew.-% in Methylenchlorid löslich. Diese Lösung ist über mehr als 100 Stunden stabil, d.h. es erfolgt kein Ausfallen des Polymeren.

II.4. Polyester

Die Herstellung des Polyesters erfolgt durch umgekehrte Grenzflächen-Polykondensation, ein Verfahren, das beispielsweise in "Methoden der organischen Chemie (Houben/Weyl), Bd. E 20 (Makromolekulare Stoffe), Stuttgart 1987, S.1420" beschrieben ist. In einem Rundkolben werden in 160 ml Methylenchlorid, 7,13 g (0,0351 Mol) Terephthalsäuredichlorid und 0,11 g (0,0005 Mol) Benzyl-triethylammoniumchlorid suspendiert. Dazu wird eine Lösung von 13,11 g (0,0350 Mol) 2,2′-Bis-(4-hydroxyphenoxy)-biphenyl in verdünnter Natronlauge (hergestelllt aus 2,99 g Natriumhydroxid und 159,42 g dest. Wasser) unter starkem Rühren im Verlauf von 30 Minuten zugetropft, wobei sich ein weisser Niederschlag bildet. Nach vollständiger Zugabe wird die Mischung weitere drei Stunden gerührt, wobei sich eine sehr viskose Suspension ergibt, die schliesslich mit Wasser verdünnt wird. Dabei setzt sich das Polymere als feiner weisser Niederschlag ab. Die überstehende Lösung wird dekantiert und der Niederschlag wird mit Aceton gewaschen.
Das isolierte Polymere wird 32 Stunden bei 150 °C im Vakuumtrockenschrank getrocknet. Es besitzt eine reduzierte Viskosität (1 % in NMP bei 25 °C) von 0,24 dl/g, eine durch DSC bestimmte Glasübergangstemperatur von 156 °C und einen Schmelzpunkt von 265 °C.

II.5. 2,2′-Bis-(4-hydroxyphenoxy)-biphenyl-diglcidylether

Zu einer Suspension von 33,3 g (0,09 Mol) 2,2′-Bis-(4-hydroxyphenoxy)-biphenyl und 116,6 g (1,26

Mol) Epichlorhydrin werden bei 60° C 0,63 g Tetramethylammoniumchlorid in 0,7 ml Wasser zugegebenen. Es wird 90 Minuten lang bei 110° C gerührt und wieder auf 60° C abgekühlt. Anschliessend werden bei 60° C und einem Innendruck von etwa 140 mbar (Wasserstrahlvakuum) eine 50 %ige wäsrige Lösung von 8,64 g (0,216 Mol) NaOH langsam zugetropft. Nach beendeter Zugabe wird 3 Stunden lang bei 60° C und einem Innendruck von etwa 150 mbar am Wasserabscheider gekocht. Nach dem Abkühlen auf Raumtemperatur wird die Reaktionslösung filtriert und der Rückstand mit 50 ml $CH_2Cl_2$ gewaschen. Die vereinigten organischen Phasen werden mit 50 ml Wasser, 50 ml 10 %iger $NH_4Cl$ und wieder mit 50 ml Wasser gewaschen, über $Na_2SO_4$ getrocknet und am Rotationsverdampfer eingedampft. Nach Trocknen am Hochvakuum (etwa $10^{-2}$ mbar) bei 70° C verbleiben 41,3 g (95 %) hellbrauner Feststoff mit einem Schmelzbereich von 100-110° C.
Epoxidgehalt (titriert): 4,08 Aequ. kg (entspricht 98,3 %)
Viskosität: 45 mPa.s bei 120° C.

II.6. Polyester

In ähnlicher Weise wie in Beispiel II.4 wird ein Polyester aus Isophtalsäuredichlorid und 2,2'-Bis(4-hydroxyphenoxy)biphenyl hergestellt.
Dazu werden in einem Rundkolben in 250 ml Methylenchlorid 7.13 g (0,0351 Mol) Isophtalsäuredichlorid und 0,23 g (0,001 Mol) Benzyltriethylammoniumchlorid suspendiert. Dazu wird eine Lösung von 13,13 g (0,0351 Mol) 2,2'-Bis(4-hydroxyphenoxy)biphenyl in verdünnter Natronlauge (hergestellt aus 3,00 g Natriumhydroxid und 251 g dest. Wasser) unter Kühlung mit einem Eis/Kochsalz-Bad im Verlauf von 30 Minuten zugetropft, wobei sich ein weisser Niederschlag bildet. Nach vollständiger Zugabe wird die Mischung 4 Stunden gerührt, dann über Nacht stehen gelassen. Das Polymere wird durch Eingiessen des Reaktionsgemisches in Wasser und Filtration erhalten. Das Produkt wird nach Waschen mit Aceton bei 100 °C im Vakuumtrockenschrank getrocknet. Es weist eine reduzierte Viskosität (1 % in NMP bei 25 °C) von 0,56 dl/g und eine Glasübergangstemperatur (DSC) von 137 °C auf.

II.7. Polyester-amid-Copolymeres

In zu Beispiel II.6. analoger Weise erhält man aus 0,03517 Mol Isophtalsäuredichlorid, 0,001 Mol Benzyltriethylammoniumchlorid, 0,0176 Mol 4-Aminophenol und 0,0176 Mol 2,2'-Bis(4-hydroxyphenoxy)-biphenyl ein Polyester-amid-Copolymeres mit einer reduzierten Viskosität von 0,61 dl/g und einer Glasübergangstemperatur von 176 °C.

II.8. Polysulfonat

In zu Beispiel II.6. analoger Weise erhält man aus 0,0351 Mol Biphenyldisulfonsäuredichlorid, 0,001 Mol Benzyltriethylammoniumchlorid und 0,0351 Mol 2,2'-Bis(4-hydroxyphenoxy)biphenyl ein Polysulfonat mit einer reduzierten Viskosität von 0,19 dl/g und einer Glasübergangstemperatur von 150 °C.

## Ansprüche

1. Im wesentlichen lineare Polymere mit einer reduzierten Viskosität von etwa 0,1 bis etwa 2,0 dl/g (gemessen an einer 1 Gew. %igen Lösung in NMP bei 25° C) enthaltend mindestens einen die Löslichkeit des Polymeren erhöhenden Anteil an Struktureinheiten Formel I

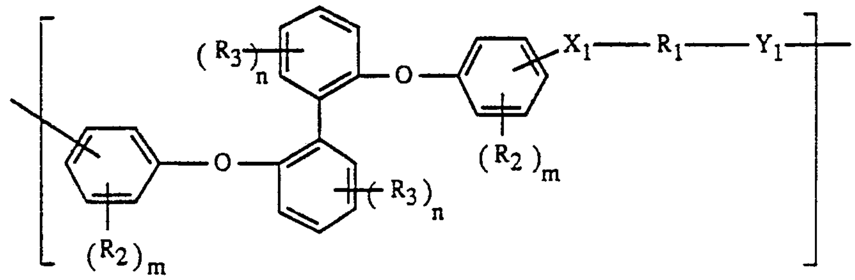

(I),

worin $X_1$ ausgewählt wird aus der Gruppe bestehend aus -O-, -O-$SO_2$-, -O-CO-, -O-CO-O-, und -O-CO-NH-, wobei der Rest $R_1$ jeweils an der rechten Seite der für $X_1$ definierten Reste gebunden ist, $Y_1$ ausgewählt wird aus der Gruppe bestehend aus -O-, -$SO_2$-O-, -CO-O-, -O-CO-O- und -NH-CO-O-, wobei der Rest $R_1$ jeweils an der linken Seite der für $Y_1$ definierten Reste gebunden ist, $R_1$ einen zweiwertigen aliphatischen, cycloaliphatischen, aromatischen oder araliphatischen Rest nach dem Entfernen der funktionellen Gruppen bedeutet, der unsubstituiert ist oder der mit ein oder mehreren Alkyl-, Alkoxy-, Alkylthio-, Alkenyl-, Cycloalkyl-, Aryl-, Cyano- oder Nitroresten substituiert ist und/oder in dem gegebenenfalls ein oder mehrere Kohlenstoffatome durch Sauerstoff-, Schwefel- und/oder Stickstoffatome ersetzt sind, oder worin die Gruppe $X_1$-$R_1$ für den Fall, dass $Y_1$ -O- oder -O-CO-O- bedeutet, auch eine direkte Bindung sein kann, $R_2$ Alkyl, Alkenyl, Cycloalkyl, Aryl oder Halogen bedeutet, $R_3$ Alkyl, Cycloalkyl, Aryl oder Halogen ist, und m und n unabhängig voneinander ganze Zahlen von 0 bis 4 darstellen.

2. Polymere gemäss Anspruch 1, dadurch gekennzeichnet, dass diese 10 bis 100 Mol%, bezogen auf das Polymere, an Struktureinheiten der Formel 1 und 90 bis 0 Mol% an Struktureinheiten der Formel II aufweisen

$\{Q\}$ (II),

worin Q sich ableitet von einem Monomeren oder einem Monomerengemisch, das sich zusammen mit den zum Aufbau der Struktureinheit der Formel I eingesetzten Monomeren copolymerisieren lässt.

3. Polymere gemäss Anspruch 2, dadurch gekennzeichnet, dass Q eine Struktureinheit der Formel III oder IV ist

$\{R_4\text{-}X_2\text{-}R_5\text{-}Y_2\}$(III),

$\{R_6\text{-}X_3\text{-}R_7\text{-}Y_3\}$(IV),

worin $R_4$ der Rest einer Dihydroxyverbindung nach dem Entfernen der funktionellen Gruppen ist, $R_6$ der Rest eines Aminoalkohols nach dem Entfernen der funktionellen Gruppen ist, $R_5$ und $R_7$ eine der für $R_1$ definierten Bedeutungen besitzen, $Y_2$und $Y_3$ eine der für $Y_1$ definierten Bedeutungen besitzen, $X_2$ eine der für $X_1$ definierten Bedeutungen besitzt, und $X_3$ ausgewählt wird aus der Gruppe bestehend aus -NH-$SO_2$-, -NH-CO-, -NH-CO-O- und -NH-CO-NH-.

4. Polymere gemäss Anspruch 3, dadurch gekennzeichnet, dass diese zu 10 bis 100 Mol% aus wiederkehrenden Struktureinheiten der Formel I und zu 90 bis 0 Mol% aus wiederkehrenden Struktureinheiten der Formel III oder IV bestehen, worin $R_1$ und $R_4$ bis R7 aromatische Reste sind.

5. Polymere gemäss einem der Ansprüche 1 bis 4, dadurch gekennzeichnet, dass diese mindestens 5 Mol %, bezogen auf das Polymere, an Resten der Formel VII enthalten

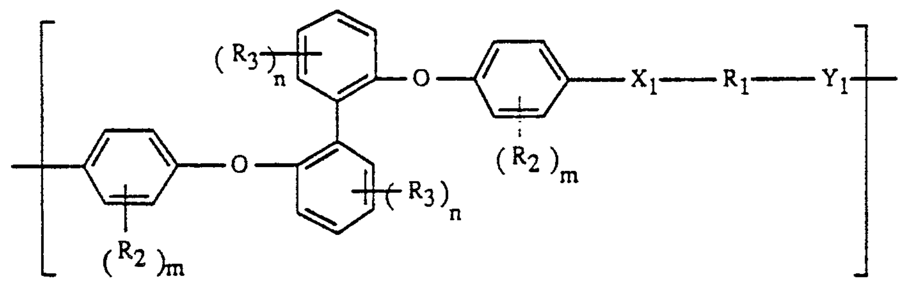

(VII),

worin $R_1$, $R_2$, $R_3$, $X_1$, $Y_1$, m und n die in Anspruch 1 definierte Bedeutung besitzen.

6. Polymere gemäss einem der Ansprüche 1 bis 5, dadurch gekennzeichnet, dass $X_1$ und $X_2$ ausgewählt werden aus der Gruppe bestehend aus -O-CO-, -O-CO-O- und insbesondere -O-, dass $X_3$ ausgewählt wird aus der Gruppe bestehend aus -NH-CO- und -NH-CO-NH-, und dass $Y_1$, $Y_2$, $Y_3$ ausgewählt werden aus der Gruppe bestehend aus -CO-O-, -O-CO-O- und insbesondere -O-.

7. Polymere gemäss Anspruch 6, dadurch gekennzeichnet, dass m und n 0 sind, $X_1$ -O-CO- oder insbesondere -O- ist, $Y_1$ -CO-O- oder insbesondere -O- ist, und $R_1$ ein zweiwertiger carbocyclisch-aromatischer Rest nach dem Entfernen der funktionellen Gruppen ist.

8. Polymere gemäss Anspruch 6, dadurch gekennzeichnet, dass diese im wesentlichen aus 5 bis 100 Mol% an wiederkehrenden Struktureinheiten der Formel Ia und aus 95 bis 0 Mol % an wiederkehrenden Struktureinheiten der Formel IIa bestehen, wobei die Prozent-angaben auf das Polymere bezogen sind

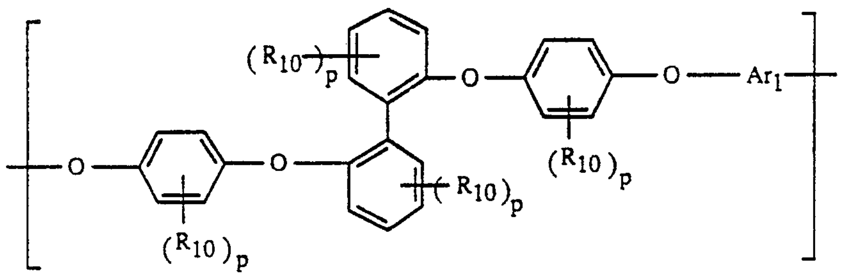
(Ia),

$-O-Ar_2-O-Ar_3-$ (IIa),

worin $Ar_1$ und $Ar_3$ unabhängig voneinander zweiwertige Reste der Formeln Va und/oder Vb sind,

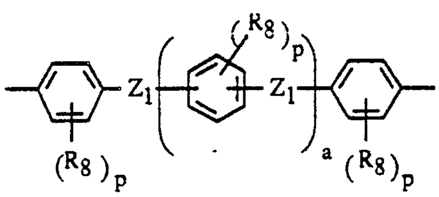
(Va),
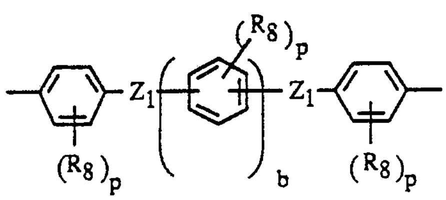
(Vb),

$Ar_2$ ein zweiwertiger Rest der Formeln VIa, VIb, VIc, VId, VIe, VIf und/oder VIg ist,

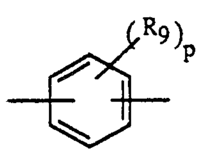
(VIa),
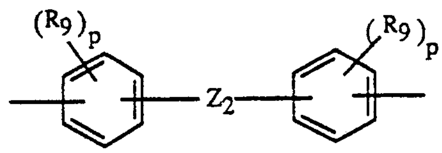
(VIb),

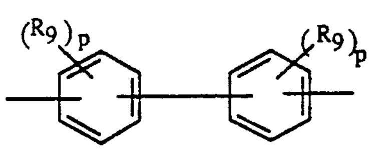
(VIc),
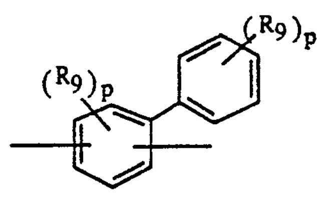
(VId),

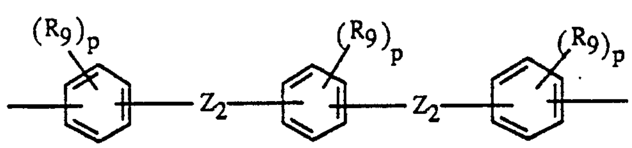
(VIe),
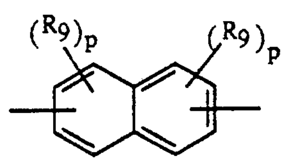
(VIf)

und
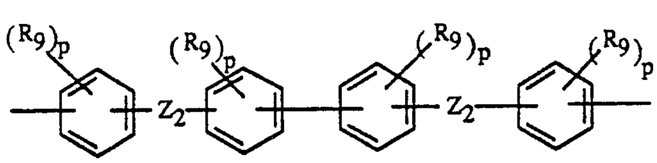
(VIg),

worin $R_8$ und $R_9$ unabhängig voneinander $C_1$-$C_6$Alkyl oder Halogen, insbesondere Chlor oder Brom bedeuten, p 1,2 oder insbesondere 0 ist, a 0, 1 oder 2 bedeutet, b 2 oder 3 ist, $Z_1$ ausgewählt wird aus der Gruppe bestehend aus -CO-, $-SO_2-$ und -SO-, $Z_2$ ausgewählt wird aus der Gruppe bestehend aus -CO-,

-SO-, $-SO_2-$, -S-, -O-, $-CH_2-$, $-C(CH_3)_2-$, $-C(CF_3)_2-$, $-C(CH_3)(C_6H_5)-$ und $-C(C_6H_5)_2-$, und $R_{10}$ $C_1$-$C_3$-Alkyl, $C_3$-$C_4$-Alkenyl, Chlor oder Brom bedeutet.

9. Polymere gemäss Anspruch 8, dadurch gekennzeichnet, dass diese im wesentlichen aus 10 bis 100 Mol% an wiederkehrenden Struktureinheiten der Formel Ia und aus 90 bis 0 Mol % an wiederkehrenden Struktureinheiten der Formel IIa bestehen, worin $Ar_1$ und $Ar_3$ ausgewählt sind aus der Gruppe bestehend aus

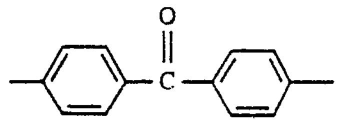 und

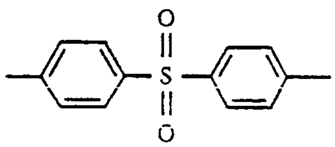 ,

und $Ar_2$ ausgewählt ist aus der Gruppe bestehend aus

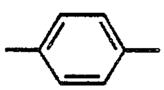 , 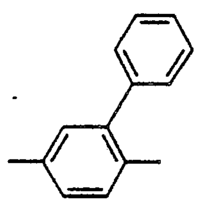 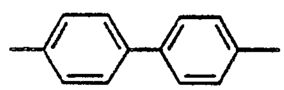 , 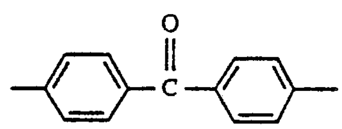 ,

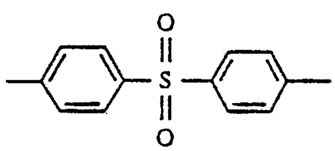 und 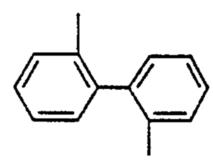 .

10. Polymere gemäss Anspruch 1, dadurch gekennzeichnet, dass diese die Struktur der Formel IX besitzen

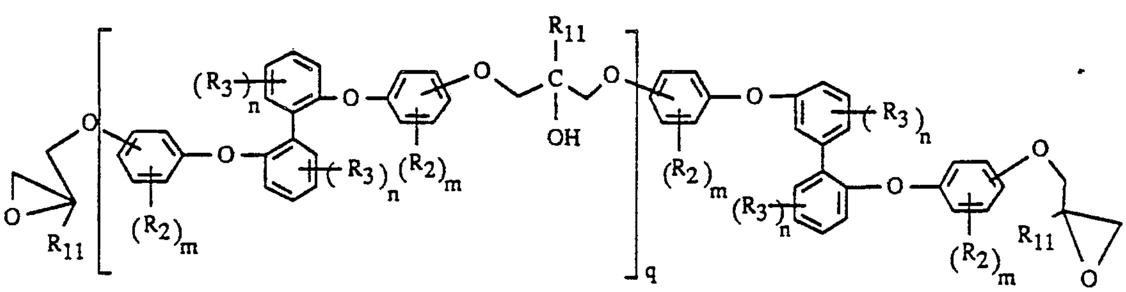

(IX),

worin $R_{11}$ $C_1$-$C_6$-Alkyl, besonders Methyl oder insbesondere Wasserstoff ist, $R_2$, $R_3$, m und n die in Anspruch 1 definierten Bedeutungen besitzen und q eine ganze Zahl von 0 bis etwa 10 ist.

11. Polymere gemäss Anspruch 1, dadurch gekennzeichnet, dass diese im wesentlichen die wiederkehrende Struktureinheit der Formel XI aufweisen

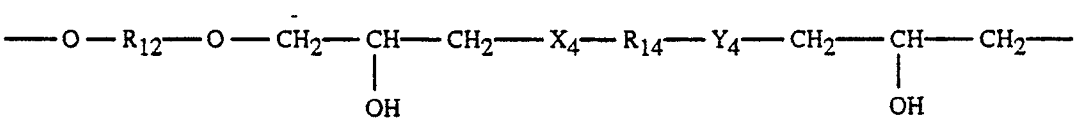

(XI),

worin $R_{12}$ der Rest eines Dialkohols nach dem Entfernen der Hydroxylgruppen ist, $X_4$ -O-CO-,-NH- oder insbesondere -O- ist, wobei $R_{14}$ jeweils an der rechten Seite der für $X_4$ definierten Reste gebunden ist, $Y_4$ -CO-O-, -NH- oder insbesondere -O- ist, wobei $R_{14}$ jeweils an der linken Seite der für $Y_4$ definierten Reste gebunden ist, $X_4$ und $Y_4$ jeweils die gleiche Bedeutung besitzen, und und $R_{14}$ der Rest eines Diglycidylesters, einer Di-(N-glycidyl)-verbindung, oder insbesondere eines Diglycidylethers nach dem Entfernen der Glycidylgruppen ist, mit der Massgabe, dass mindestens ein Teil der Reste $R_{12}$ eine Struktur der Formel

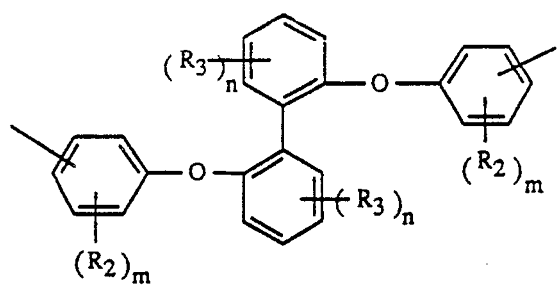

aufweist, worin $R_2$, $R_3$, m und n die in Anspruch 1 definierte Bedeutung besitzen.

12. Polymere gemäss Anspruch 11, dadurch gekennzeichnet, dass diese im wesentlichen aus den wiederkehrenden Struktureinheiten der Formel XII bestehen

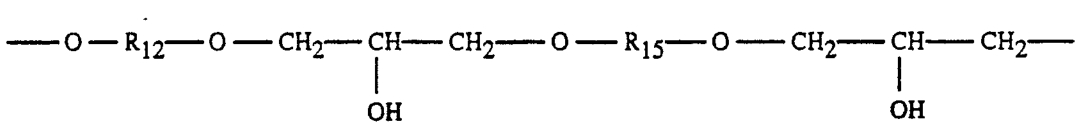

(XII),

worin $R_{12}$ die in Anspruch 11 definierte Bedeutung besitzt und $R_{15}$ der Rest eines Diglycidylethers, vorzugsweise eines Diglycidylethers auf Basis eines Bisphenols, nach dem Entfernen der Glycidylethergruppen ist, mit der Massgabe, dass mindestens ein Teil der Reste $R_{12}$ und/oder $R_{15}$ eine Struktur der Formel

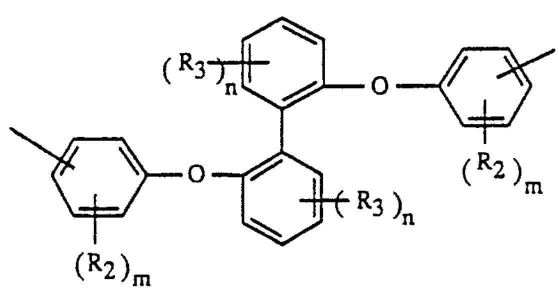

aufweist, worin $R_2$, $R_3$, m und n die in Anspruch 1 definierte Bedeutung besitzen.

13. Lösung enthaltend etwa 1 bis 75 Gew.%, bezogen auf die Lösung, eines Polymeren gemäss Anspruch 1 gelöst in einem organischen Lösungsmittel.

14. Verbindungen der Formel VIII

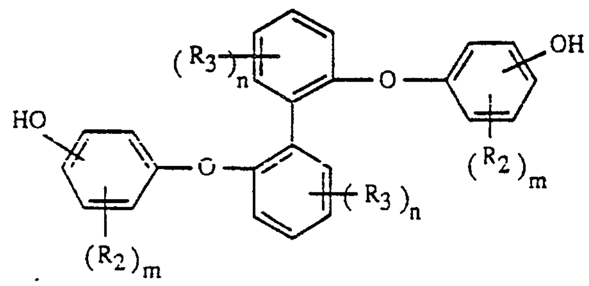

(VIII),

worin $R_2$, $R_3$, m und n die in Anspruch 1 definierte Bedeutung besitzen.

15. Verbindungen der Formel VIII gemäss Anspruch 14, dadurch gekennzeichnet, dass die Hydroxylgruppen sich jeweils in 4-Position zur O-Brücke befinden.

16. Verbindungen der Formel VIII gemäss Anspruch 14, dadurch gekennzeichnet, dass $R_2$ und $R_3$ unabhängig voneinander Alkyl oder Halogen sind und m und n jeweils 1,2 oder insbesondere 0 bedeuten.

17. Verwendung von Bisphenolen der Formel VIII gemäss Anspruch 14 als Monomerkomponente zur Herstellung von Polymeren.

18. Verwendung der Polymeren gemäss einem der Ansprüche 1 bis 12 zur Herstellung von Formkörpern oder Folien, als Matrixharze, Klebstoffe oder Ueberzugsmittel, oder als Zusätze zu anderen Polymeren.

Patentansprüche für folgenden Vertragsstaat: ES

1. Verfahren zur Herstellung von im wesentlichen linearen Polymeren mit einer reduzierten Viskosität von etwa 0,1 bis etwa 2,0 dl/g (gemessen an einer 1 Gew. %igen Lösung in NMP bei 25 °C) enthaltend mindestens einen die Löslichkeit des Polymeren erhöhenden Anteil an Struktureinheiten Formel I

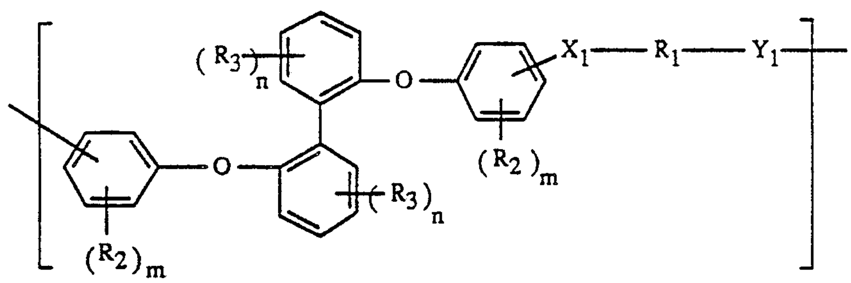

(I),

worin $X_1$ ausgewählt wird aus der Gruppe bestehend aus -O-, -O-$SO_2$-, -O-CO-, -O-CO-O-, und -O-CO-NH-, wobei der Rest $R_1$ jeweils an der rechten Seite der für $X_1$ definierten Reste gebunden ist, $Y_1$ ausgewählt wird aus der Gruppe bestehend aus -O-, -$SO_2$-O-, -CO-O-, -O-CO-O- und -NH-CO-O-, wobei der Rest $R_1$ jeweils an der linken Seite der für $Y_1$ definierten Reste gebunden ist, $R_1$ einen zweiwertigen aliphatischen, cycloaliphatischen, aromatischen oder araliphatischen Rest nach dem Entfernen der funktionellen Gruppen bedeutet, der unsubstituiert ist oder der mit ein oder mehreren Alkyl-, Alkoxy-, Alkylthio-, Alkenyl-, Cycloalkyl-, Aryl-, Cyano- oder Nitroresten substituiert ist und/oder in dem gegebenenfalls ein oder mehrere Kohlenstoffatome durch Sauerstoff-, Schwefel- und/oder Stickstoffatome ersetzt sind, oder worin die Gruppe $X_1$-$R_1$ für den Fall, dass $Y_1$ -O- oder -O-CO-O- bedeutet, auch eine direkte Bindung sein kann, $R_2$ Alkyl, Alkenyl, Cycloalkyl, Aryl oder Halogen bedeutet, $R_3$ Alkyl, Cycloalkyl, Aryl oder Halogen ist, und m und n unabhängig voneinander ganze Zahlen von 0 bis 4 darstellen,
dadurch gekennzeichnet, dass man ein Bisphenol der Formel VIII

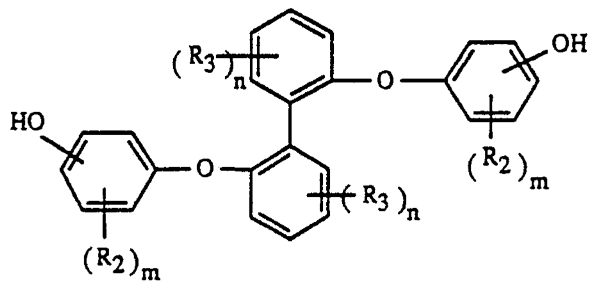

(VIII),

worin $R_2$, $R_3$, m und n die oben angegebene Bedeutung haben, in Gegenwart einer oder mehrerer mit dem Bisphenol der Formel VIII copolymerisierbarer Monomerer der Formel XIII gegebenenfalls zusammen mit weiteren Dihydroxyverbindungen der Formel XIV und/oder Aminoalkoholen der Formel XV umsetzt

$FG-R_1-FG$ (XIII), $HO-R_4-OH$ (XIV), $H_2N-R_6-OH$ (XV),

worin $R_1$ und $R_4$ die oben definierte Bedeutung besitzen, $R_6$ der Rest eines Aminoalkohols nach dem Entfernen der funktionellen Gruppen ist und FG eine funktionelle Gruppe ist, die zur Umsetzung mit einer Hydroxyl- oder einer primären Aminogruppe befähigt ist.

2. Verfahren zur Herstellung von Polymeren gemäss Anspruch 1, dadurch gekennzeichnet, dass diese 10 bis 100 Mo1%, bezogen auf das Polymere, an Struktureinheiten der Formel I und 90 bis 0 Mol% an Struktureinheiten der Formel II aufweisen

$\{Q\}$ (II),

worin Q sich ableitet von einem Monomeren oder einem Monomerengemisch, das sich zusammen mit den zum Aufbau der Struktureinheit der Formel I eingesetzten Monomeren copolymerisieren lässt.

3. Verfahren zur Herstellung von Polymeren gemäss Anspruch 2, dadurch gekennzeichnet, dass Q eine Struktureinheit der Formel III oder IV ist

$\{R_4-X_2-R_5-Y_2\}$ (III),

$\{R_6-X_3-R_7-Y_3\}$ (IV),

worin $R_4$ der Rest einer Dihydroxyverbindung nach dem Entfernen der funktionellen Gruppen ist, $R_6$ der Rest eines Aminoalkohols nach dem Entfernen der funktionellen Gruppen ist, $R_5$ und $R_7$ eine der für $R_1$ definierten Bedeutungen besitzen, $Y_2$ und $Y_3$ eine der für $Y_1$ definierten Bedeutungen besitzen, $X_2$ eine der für $X_1$ definierten Bedeutungen besitzt, und $X_3$ ausgewählt wird aus der Gruppe bestehend aus $-NH-SO_2-$, -NH-CO-, -NH-CO-O- und -NH-CO-NH-.

4. Verfahren zur Herstellung von Polymeren gemäss Anspruch 3, dadurch gekennzeichnet, dass diese zu 10 bis 100 Mol% aus wiederkehrenden Struktureinheiten der Formel I und zu 90 bis 0 Mol% aus wiederkehrenden Struktureinheiten der Formel III oder IV bestehen, worin $R_1$ und $R_4$ bis $R_7$ aromatische Reste sind.

5. Verfahren zur Herstellung von Polymeren gemäss einem der Ansprüche 1 bis 4, dadurch gekennzeichnet, dass diese mindestens 5 Mol %, bezogen auf das Polymere, an Resten der Formel VII enthalten

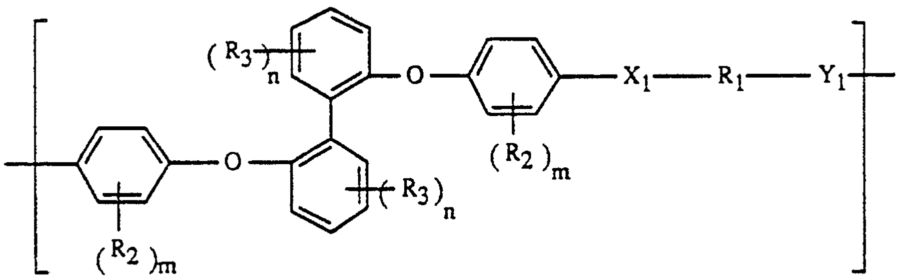

(VII),

worin $R_1$, $R_2$, $R_3$, $X_1$, $Y_1$, m und n die in Anspruch 1 definierte Bedeutung besitzen.

6. Verfahren zur Herstellung von Polymeren gemäss einem der Ansprüche 1 bis 5, dadurch gekennzeichnet, dass $X_1$ und $X_2$ ausgewählt werden aus der Gruppe bestehend aus -O-CO-, -O-CO-O- und insbesondere -O-, dass $X_3$ ausgewählt wird aus der Gruppe bestehend aus -NH-CO- und -NH-CO-NH-, und dass $Y_1$, $Y_2$, $Y_3$ ausgewählt werden aus der Gruppe bestehend aus -CO-O-, -O-CO-O- und insbesondere -O-.

7. Verfahren zur Herstellung von Polymeren gemäss Anspruch 6, dadurch gekennzeichnet, dass m und n 0 sind, $X_1$ -O-CO- oder insbesondere -O- ist, $Y_1$ -CO-O- oder insbesondere -O- ist, und $R_1$ ein zweiwertiger carbocyclisch-aromatischer Rest nach dem Entfernen der funktionellen Gruppen ist.

8. Verfahren zur Herstellung von Polymeren gemäss Anspruch 6, dadurch gekennzeichnet, dass diese im wesentlichen aus 5 bis 100 Mol% an wiederkehrenden Struktureinheiten der Formel Ia und aus 95 bis 0 Mol % an wiederkehrenden Struktureinheiten der Formel IIa bestehen, wobei die Prozentangaben auf das Polymere bezogen sind

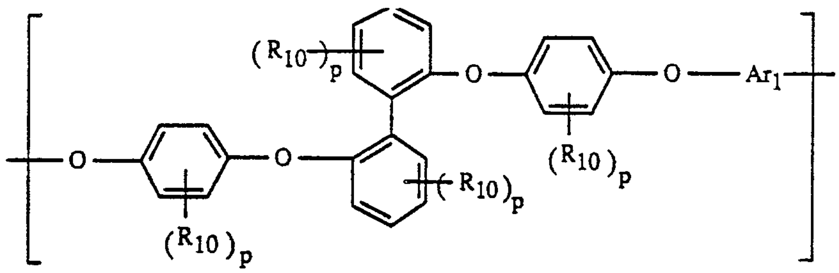 (Ia),

$$-O-Ar_2-O-Ar_3- \quad \text{(IIa)},$$

worin $Ar_1$ und $Ar_3$ unabhängig voneinander zweiwertige Reste der Formeln Va und/oder Vb sind,

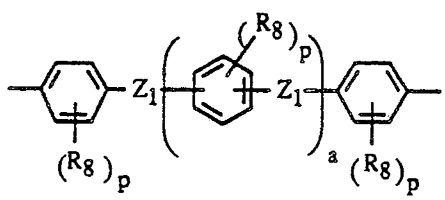 (Va), 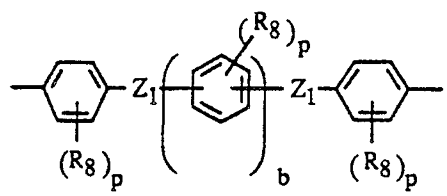 (Vb),

$Ar_2$ ein zweiwertiger Rest der Formeln VIa, VIb, VIc, VId, VIe, VIf und/oder VIg ist,

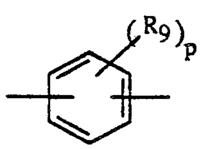 (VIa), 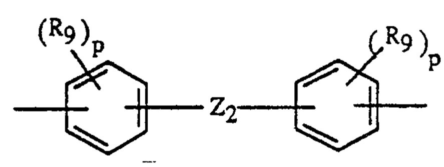 (VIb),

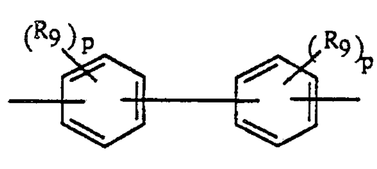 (VIc), 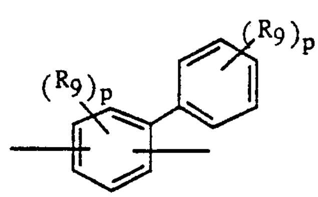 (VId),

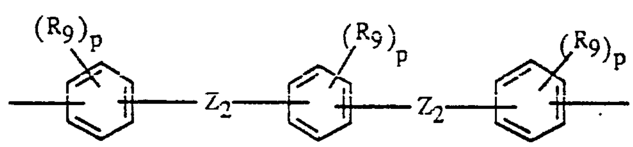 (VIe), 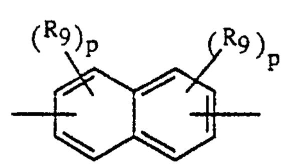 (VIf)

und 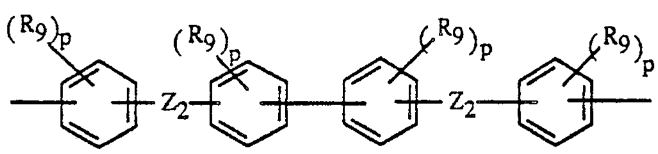 (VIg),

worin $R_8$ und $R_9$ unabhängig voneinander $C_1$-$C_6$Alkyl oder Halogen, insbesondere Chlor oder Brom bedeuten, p 1, 2 oder insbesondere 0 ist, a 0, 1 oder 2 bedeutet, b 2 oder 3 ist, $Z_1$ ausgewählt wird aus der Gruppe bestehend aus -CO-, $-SO_2-$ und -SO-) $Z_2$ ausgewählt wird aus der Gruppe bestehend aus -CO-, -SO-, $-SO_2-$, -S-, -O-, $-CH_2-$) $-C(CH_3)_2-$, $-C(CF_3)_2-$, $-C(CH_3)(C_6H_5)-$ und $-C(C_6H_5)_2-$, und $R_{10}$ $C_1$-$C_3$-Alkyl,

$C_3$-$C_4$-Alkenyl, Chlor oder Brom bedeutet.

9. Verfahren zur Herstellung von Polymeren gemäss Anspruch 8, dadurch gekennzeichnet, dass diese im wesentlichen aus 10 bis 100 Mol% an wiederkehrenden Struktureinheiten der Formel Ia und aus 90 bis 0 % Mol an wiederkehrenden Struktureinheiten der Formel IIa bestehen, worin $Ar_1$ und $Ar_3$ ausgewählt sind aus der Gruppe bestehend aus

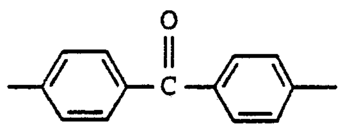 und 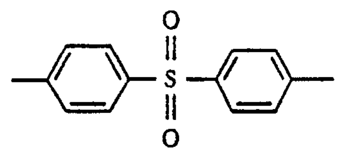 ,

und $Ar_2$ ausgewählt ist aus der Gruppe bestehend aus

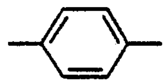 , 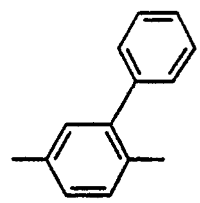 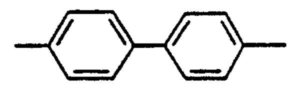 ,

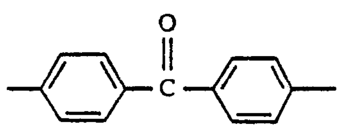 , 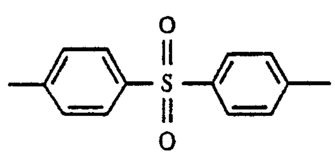 und 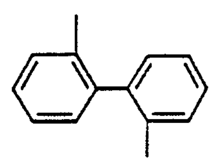 .

10. Verfahren zur Herstellung von Polymeren gemäss Anspruch 1, dadurch gekennzeichnet, dass diese die Struktur der Formel IX besitzen

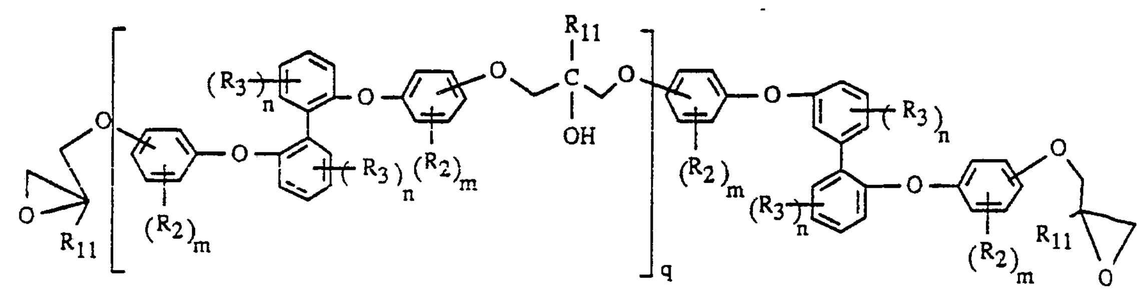

(IX),

worin $R_{11}$ $C_1$-$C_6$-Alkyl, besonders Methyl oder insbesondere Wasserstoff ist, $R_2$, $R_3$, m und n die in Anspruch 1 definierten Bedeutungen besitzen und q eine ganze Zahl von 0 bis etwa 10 ist.

11. Verfahren zur Herstellung von Polymeren gemäss Anspruch 1, dadurch gekennzeichnet, dass diese im wesentlichen die wiederkehrende Struktureinheit der Formel XI aufweisen

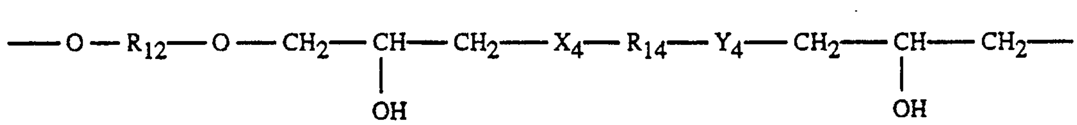

(XI),

worin $R_{12}$ der Rest eines Dialkohols nach dem Entfernen der Hydroxylgruppen ist, $X_4$ -O-CO-,-NH- oder insbesondere -O- ist, wobei $R_{14}$ jeweils an der rechten Seite der für $X_4$ definierten Reste gebunden ist, $Y_4$ -

CO-O-, -NH- oder insbesondere -O- ist, wobei $R_{14}$ jeweils an der linken Seite der für $Y_4$ definierten Reste gebunden ist, $X_4$ und $Y_4$ jeweils die gleiche Bedeutung besitzen, und und $R_{14}$ der Rest eines Diglycidylesters, einer Di-(N-glycidyl)-verbindung, oder insbesondere eines Diglycidylethers nach dem Entfernen der Glycidylgruppen ist, mit der Massgabe, dass mindestens ein Teil der Reste $R_{12}$ eine Struktur der Formel

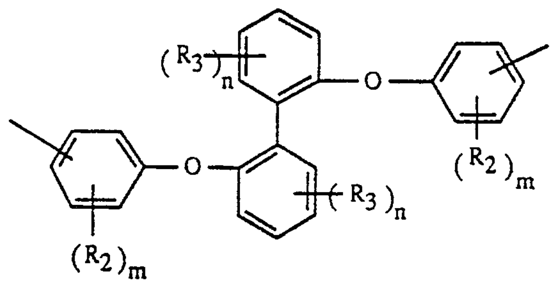

aufweist, worin $R_2$, $R_3$, m und n die in Anspruch 1 definierte Bedeutung besitzen.

12. Verfahren zur Herstellung von Polymeren gemäss Anspruch 11, dadurch gekennzeichnet, dass diese im wesentlichen aus den wiederkehrenden Struktureinheiten der Formel XII bestehen

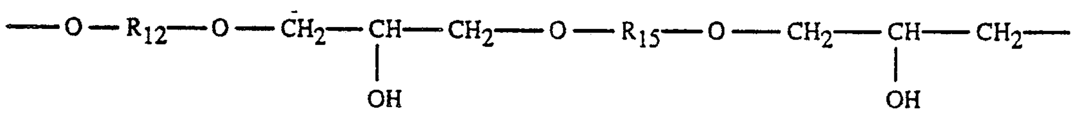

(XII),

worin $R_{12}$ die in Anspruch 11 definierte Bedeutung besitzt und $R_{15}$ der Rest eines Diglycidylethers, vorzugsweise eines Diglycidylethers auf Basis eines Bisphenols, nach dem Entfernen der Glycidylethergruppen ist, mit der Massgabe, dass mindestens ein Teil der Reste $R_{12}$ und/oder $R_{15}$ eine Struktur der Formel

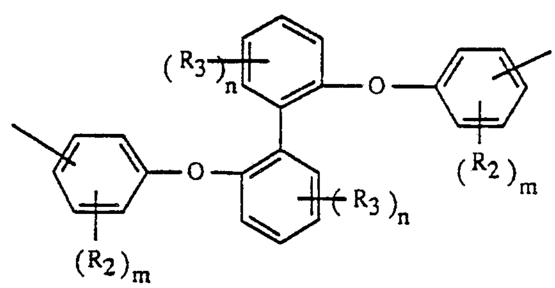

aufweist, worin $R_2$, $R_3$, m und n die in Anspruch 1 definierte Bedeutung besitzen.

13. Lösung enthaltend etwa 1 bis 75 Gew.%, bezogen auf die Lösung eines Polymeren gemäss Anspruch 1 gelöst in einem organischen Lösungsmittel.

14. Verfahren zur Herstellung von Verbindungen der Formel VIII

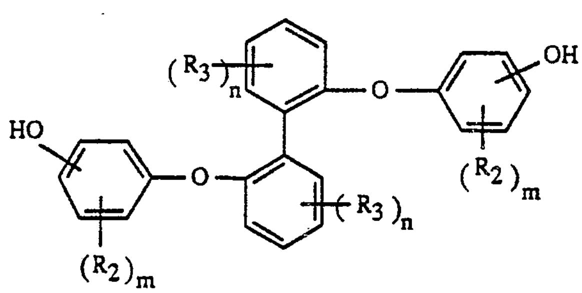

(VIII),

worin $R_2$, $R_3$, m und n die in Anspruch 1 definierte Bedeutung besitzen, dadurch gekennzeichnet, dass man

2,2'-Dihydroxybiphenyl mit etwa der doppelt stöchiometrischen Menge eines Halogenacetophenons, beispielsweise mit 2-, 3- oder 4-Fluoracetophenon, umsetzt, das so erhaltene Bis-acetylphenoxybiphenyl durch Baeyer-Villiger Oxidation in das entsprechende Bis-acetoxyphenoxybiphenyl überführt und in einer Folgeoperation zum Produkt der Formel VIII verseift.

15. Verfahren zur Herstellung von Verbindungen der Formel VIII gemäss Anspruch 14, dadurch gekennzeichnet, dass die Hydroxylgruppen sich jeweils in 4-Position zur O-Brücke befinden.

16. Verfahren zur Herstellung von Verbindungen der Formel VIII gemäss Anspruch 14, dadurch gekennzeichnet, dass $R_2$ und $R_3$ unabhängig voneinander Alkyl oder Halogen sind und m und n jeweils 1,2 oder insbesondere 0 bedeuten.

17. Verwendung der Polymeren gemäss einem der Ansprüche 1 bis 12 zur Herstellung von Formkörpern oder Folien, als Matrixharze, Klebstoffe oder Ueberzugsmittel, oder als Zusätze zu anderen Polymeren.